(19) Europäisches Patentamt
European Patent Office
Office européen des brevets

(11) Numéro de publication: **0 357 495**
**A2**

# (12) DEMANDE DE BREVET EUROPEEN

(21) Numéro de dépôt: **89402340.7**

(22) Date de dépôt: **24.08.89**

(51) Int. Cl.[5]: **C 07 H 19/073**
A 61 K 31/70

(30) Priorité: **26.08.88 FR 8811284**

(43) Date de publication de la demande:
**07.03.90 Bulletin 90/10**

(84) Etats contractants désignés:
**AT BE CH DE ES FR GB GR IT LI LU NL SE**

(71) Demandeur: **IRE-CELLTARG S.A.**
**Zone Industrielle**
**B-6220 Fleurus (BE)**

(72) Inventeur: **De Bethune, Marie-Pierre**
**15, Twee Leeuwenstraat**
**B-3078 Everberg (BE)**

**De Clercq, Erik Désiré Alice**
**35/7 Paul Lebrunstraat**
**B-3000 Leuven (BE)**

**DeJonghe, Jean-Paul**
**Rue Ch. Jaumotte, 94**
**B-1350 Wavre (BE)**

**Pauwels, Rudi Wilfried Jan**
**Minnezangerslaan, 109**
**B-1080 Brussels (BE)**

**Trouet, André**
**Predikherenberg, 20**
**B-3009 Winksele (BE)**

(74) Mandataire: **Martin, Jean-Jacques et al**
**Cabinet REGIMBEAU 26, Avenue Kléber**
**F-75116 Paris (FR)**

## (54) Dérivés de la 3'azido-3' déoxythymidine (AZT) actifs contre le virus du sida.

(57) L'invention a pour objet des composés chimiques dérivés de la 3'-azido-3' déoxythymidine et leurs sels pharmaceutiquement acceptables, caractérisés en ce qu'ils répondent à la formule générale I

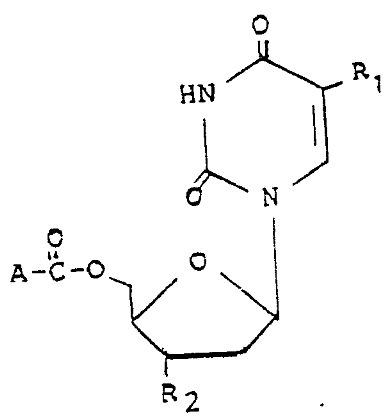

I

dans laquelle
. $R_1$ représente H, un radical alkyle inférieur, un radical alcoxy inférieur, un radical hydroxyalcoyle inférieur ou un halogène. .
$R_2$ est $N_3$ ou un radical -CN et
. A représente un radical hydrocarboné éventuellement substitué, portant au moins une fonction polaire, notamment carboxylique, sulfonique, ou amine éventuellement protégée, autre que le groupe hémisuccinyl.

La présente invention a également pour objet des compositions pharmaceutiques comprenant ces composés ou le dérivé hémisuccinyl en position 5' de l'AZT ainsi que des procédés de préparation desdits composés.

EP 0 357 495 A2

## Description

### DERIVES DE LA 3′ AZIDO-3′ DEOXYTHYMIDINE (AZT) ACTIFS CONTRE LE VIRUS DU SIDA

La présente invention concerne de nouveaux composés chimiques dérivés de la 3′azido-3′déoxythymidine, ainsi que des compositions pharmaceutiques en comprenant.

Le SIDA ou syndrome d'immunodéficience acquise est causé par un rétrovirus, le HIV (Human Immunodeficiency Virus), qui se transmet par contact sanguin ou sexuel.

L'infection est généralement suivie de symptomes du type mononucléose. Le virus se multiplie activement dans l'organisme jusqu'à l'apparition d'anticorps (séroconversion). Suit alors une période de latence qui peut varier de quelques mois à plusieurs années, période au cours de laquelle le système immunitaire se dégrade progressivement, tandis que le virus vraisemblablement se multiplie très lentement, de manière non détectable cliniquement. L'évolution vers la maladie est caractérisée par l'apparition d'une lymphadénopathie progressive généralisée (PGL) accompagnée d'autres manifestations cliniques : anergie, perte de poids, diminution du nombre de lymphocytes CD4 entre autres. Le dernier stade, celui du SIDA proprement dit, est caractérisé d'une part par une reprise de la multiplication active du virus accompagnée de la disparition de certains anticorps, et d'autre part, par une série d'infections opportunistes (virales, bactériennes, mycotiques) et de cancers (sarcome de Kaposi essentiellement) menant à une issue fatale à plus ou moins brève échéance.

Plusieurs types cellulaires peuvent être infectés par le HIV dans l'organisme, mais les deux principaux sont d'une part, les lymphocytes CD4 qui jouent le rôle de stimulateurs des lymphocytes B et des macrophages, et d'autre part, les macrophages eux-mêmes. Alors que la multiplication du virus dans les premiers entraine la mort des cellules, il semble que les macrophages constituent un réservoir de virus que, de part leur fonction de destruction de foyers infectieux, ils disséminent dans plusieurs organes, y compris le cerveau.

Beaucoup de produits ont été proposés et sont testés comme agents capable de soigner cette maladie. Parmi ceux-ci, les seuls à montrer une efficacité significative sont certains analogues de nucléosides. Plus particulièrement, l'AZT ou 3′azido-3′déoxythymidine donne des résultats intéressants. Il s'agit d'un inhibiteur de la réverse transcriptase, c'est-à-dire l'enzyme du virus capable de transcrire son génome de RNA en DNA qui sera intégré dans le génome cellulaire.

Administré à des patients au stade SIDA, l'AZT entraine une diminution de la multiplication du virus, une légère augmentation du nombre de lymphocytes CD4, une reprise de poids et une amélioration sensible de l'état général. De plus, l'AZT traverse la barrière hémato-encéphalique et a un effet bénéfique dans le cas de démences associées à l'infection par le HIV. Ces manifestations positives sont toutefois réversibles, et on estime actuellement à environ 18 mois l'augmentation du temps de survie des patients traités. En outre, et surtout, l'utilisation de l'AZT est limitée par sa forte toxicité médullaire, essentiellement l'anémie.

Il apparaît également, au regard de l'évolution de la maladie, qu'il serait plus opportun de traiter les personnes infectées avant que le virus ne se multiplie de manière trop importante, et n'entraine par là un déséquilibre irréversible du système immunitaire.

Toutefois, l'administration prolongée d'AZT est difficilement réalisable en raison de sa toxicité à long terme pour les cellules hématopoïétiques.

En revanche, l'administration prolongée de ces mêmes drogues, mais sous des formes réduisant fortement leur toxicité ou augmentant leur activité, est envisageable.

Le but de la présente invention est précisément de fournir de nouveaux dérivés d'une molécule active contre le virus du SIDA, l'AZT, ces nouveaux dérivés présentant un indice de sélectivité amélioré par rapport à celui de l'AZT de manière à permettre le traitement à long terme, et notamment le traitement de personnes infectées par le virus dès avant que la maladie ne se soit déclarée.

Pour ce faire, la présente invention a pour objet des esters en positions 5′ de l'AZT, le groupe en position 5′ étant lié à l'AZT par un lien ester et contenant au moins une fonction polaire notamment de type carboxylique, sulfonique, ou amine éventuellement protégée.

Certains dérivés de l'AZT selon l'invention présentent une bonne stabilité en présence de plasma humain 37°C et relarguent très lentement de l'AZT, ce qui a pour effet de diminuer la toxicité des produits.

D'autres dérivés de l'AZT selon l'invention se montrent plus actifs que l'AZT tout en ayant une toxicité comparable à celle de ce produit.

La préparation d'un dérivé hémisuccinate en position 5′ de l'AZT a été décrite dans la demande de brevet européen EP 199 451, le produit étant proposé à titre d'ester pharmaceutiquement acceptable comme précurseur de l'AZT. Toutefois, l'utilisation thérapeutique avantageuse conformément au but de la présente invention de ce dérivé 5′ hémisuccinyl de l'AZT, n'a été ni décrite ni suggérée dans la demande de brevet EP 199 451.

La présente invention a donc plus précisément pour objet de nouveaux composés chimiques, dérivés de la 3′azido-3′déoxythymidine répondant à la formule générale I et leurs sels pharmaceutiquement acceptables :

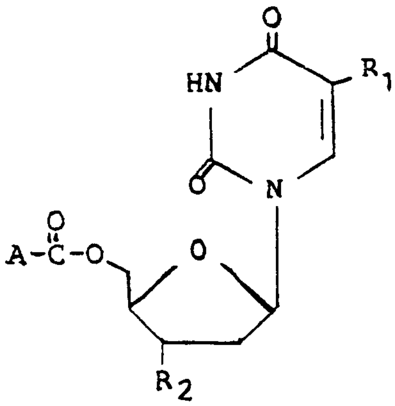

I

formule dans laquelle

. $R_1$ représente H, un radical alkyle inférieur, un radical alcoxy inférieur, un radical hydroxyalcoyle inférieur ou un halogène.

. $R_2$ est choisi parmi $N_3$ ou un radical -CN

. A représente un radical hydrocarboné éventuellement substitué portant au moins une fonction polaire telle que carboxylique, sulfonique ou amine éventuellement protégée ou telles que leurs fonctions dérivées par substitution d'hydrogène par un radical hydrocarboné inférieur qui peut, pour accentuer le caractère polaire, être raccordé à A en formant un hétérocycle, A n'étant pas le groupe hémisuccinyl.

Dans la définition de $R_1$, on entend par radical inférieur un radical comportant de 1 à 5 atomes de carbone.

Dans la définition de A, on entend par radical hydrocarboné un radical comportant de 1 à 20 atomes de carbone, le radical pouvant être éventuellement substitué, notamment par des radicaux alkyles, alcoxy alkyles, aryl, aralkyles en C1 à C7.

Bien entendu, parmi les composés de formule I il faut citer plus particulièrement les dérivés de l'AZT dans lesquels $R_1$ est égal à $CH_3$ et $R_2$ à $N_3$.

Dans un mode particulier de réalisation de l'invention

$$A-\overset{O}{\overset{\|}{C}}- \text{ représente } X-(\underset{R_3}{\underset{|}{C}}H)_n-\overset{O}{\overset{\|}{C}}-$$

où n est un nombre entier compris entre 1 et 5

$R_3$ peut être identique ou différent et représente H ou un radical hydrocarboné, tel qu'un alkyle, un aralkyle, un alcoxyalkyle, éventuellement substitués ; la chaîne latérale $R_3$ en alpha de la fonction amino d'une acide aminé $R_3$-$CH(NH_2)COOH$ entrant dans la constitution des protéines naturelles ; ou un groupe NHR avec R = H, alkyl, alcoxycarbonyl, formyl ou acyl ; ou avec un autre $R_3$ adjacent peut représenter simplement une liaison pour former une double liaison ; et X = COOH, $SO_3H$, $NH_2$ éventuellement protégée.

De préférence, X = COOH, ou $NH_2$ éventuellement protégée.

On peut citer plus particulièrement les cas : où X = COOH, $R_3$ = H

On peut citer également le cas où

$A-\overset{O}{\overset{\|}{C}}$ représente

$$RHN-\overset{COOH}{\overset{|}{C}}H-CH_2-\overset{O}{\overset{\|}{C}}- \text{ ou}$$

$$HOOC-CH_2-\underset{NHR}{\underset{|}{C}}H-\overset{O}{\overset{\|}{C}}-$$

CHE

avec R = H, alkyl, formyl, acyl, ou alcoxycarbonyl.

Une autre famille de composés selon l'invention est représentée par les composés dans lesquels

$A-\overset{O}{\overset{\|}{C}}$ représente un reste éventuellement protégé d'acide aminé A-COOH, notamment un acide aminé entrant dans la constitution des protéines naturelles.

En particulier, on peut citer les composés où dans la formule

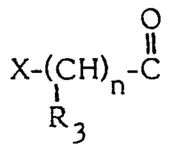

n = 1, X = $NH_2$ éventuellement protégée et $R_3$ représente la chaîne latérale en alpha de la fonction amino d'un acide aminé, notamment un acide aminé entrant dans la constitution des protéines naturelles.

Dans certains cas, dans la formule qui précède X = NH et forme également un cycle avec $R_3$ notamment lorsque A-COOH représente l'acide L-pyroglutamique.

Comme groupe protecteur de la fonction $NH_2$, on peut citer à titre illustratif et non limitatif les groupes tBoc ou Fmoc.

Les composés de formule I peuvent être préparés en faisant réagir les composés de formule II suivante

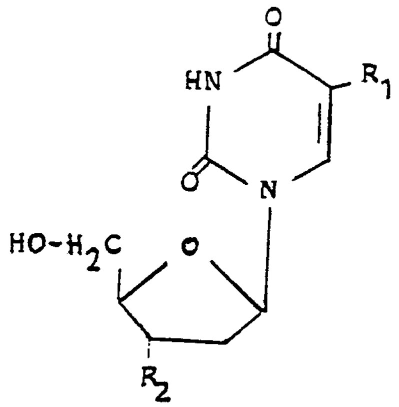

II

avec un ester d'acide carboxylique de formule

A-COOH

ou un sel halogénure de cet acide, tel qu'un chlorure ou encore un anhydride d'acide, externe ou interne, lorsque A inclut une fonction carboxylique. Le cas échéant, on aura bloqué les autres positions réactives des composés de formule II.

Ces réactions sont bien connues de l'homme de l'art.

La synthèse de l'AZT (composés de formule II) a été décrite par exemple dans l'article de DRUGS OF THE FUTURE, Volume 11, n° 12, 1986, mais également dans de nombreux autres documents de l'état de la technique.

La présente invention a donc également pour objet des compositions pharmaceutiques caractérisées en ce qu'elle comporte à titre de substance active un composé de formule I selon l'invention ou le dérivé 5'hémisuccinyl de l'AZT.

La présente invention a en outre pour objet des compositions pharmaceutiques à toxicité réduite ou activité thérapeutique augmentée par rapport aux compositions pharmaceutiques dont la substance active est l'AZT, utiles pour le traitement à long terme du SIDA, et notamment pour le traitement des personnes séropositives infectées par le virus du SIDA mais qui sont au stade asymptomatique, c'est-à-dire dont la maladie ne s'est pas encore déclarée, caractérisées en ce qu'elles comportent à titre de substance active une quantité thérapeutiquement efficace d'un composé de formule I selon l'invention ou du dérivé hémisuccinyl en position 5′ de l'AZT.

Les compositions pharmaceutiques selon l'invention peuvent contenir une quantité molaire de substances actives inférieure à la quantité d'AZT nécessaire pour obtenir une même activité thérapeutique ou une quantité molaire de substance active supérieure à la quantité d'AZT induisant une même toxicité.

La présente invention a également pour objet l'utilisation des dérivés de formule I selon l'invention ou du dérivé hémisuccinyl en position 5′ de l'AZT pour la préparation d'une composition pharmaceutique à toxicité réduite ou à activité thérapeutique augmentée pour le traitement à long terme du SIDA, et notamment avant que la maladie ne se soit déclarée.

Enfin, la présente invention a pour objet un procédé pour diminuer la toxicité ou augmenter l'activité de l'AZT, et ainsi améliorer l'indice de sélectivité de l'AZT, caractérisé en ce qu'on effectue une estérification en position 5′ d'un composé de formule II tel que l'AZT avec un acide approprié A-COOH ou un sel halogénure de cet acide, tel qu'un chlorure ou encore un anhydryde d'acide, externe ou interne, lorsque A inclut une fonction carboxylique, pour obtenir un ester d'acide carboxylique selon l'invention de formule I, ou le dérivé hémisuccinyl en position 5′ de l'AZT.

On a découvert en effet selon l'invention que le dérivé hémisuccinyl en 5′ de l'AZT montre une activité comparable à celle de l'AZT tout en ayant une toxicité considérablement inférieure, ce qui lui confère un indice de sélectivité de l'ordre de 28 000, alors que l'indice de sélectivité de l'AZT et de l'ordre de 6000.

D'autres dérivés selon l'invention comme le dérivé 5'-tBoc-valine de l'AZT, ou encore le dérivé 5'-hémiaspartyl de l'AZT, sont également moins toxiques que l'AZT avec des activités comparables. D'autre part, le dérive 5'-valine de l'AZT a une toxicité comparable à celle de l'AZT mais est plus actif que l'AZT.

D'autres avantages et caractéristiques de la présente invention apparaîtront à la lumière des exemples non limitatifs qui vont suivre.

Les figures 1 à 7 représentent des courbes de stabilité de composés selon l'invention en fonction du pH (figures 1 à 3), en présence d'enzymes lysosomiales (figure 4), en présence de sérum et de plasma (figures 5 à 7).

L'exemple 10 fait référence aux figures 8 à 11.

Les figures 8 et 9 représentent des courbes de survie des souris infectées par le virus MSV et traitées par l'AZT (figure 8) et par son dérivé hémisuccinate selon l'invention (figure 9).

aux figure 8 :
o = contrôle
● = 5 mg/kg/jour d'AZT
▲ = 25 mg/kg/jour d'AZT
■ = 125 mg/kg/jour d'AZT

figure 9 :
o = contrôle
● = 6,8 mg/kg/jour de 5'hémisuccinyl-AZT
▲ = 34 mg/kg/jour de 5'hémisuccinyl-AZT
■ = 170 mg/kg/jour de 5'hémisuccinyl-AZT

Les figures 10 et 11 représentent des courbes d'apparition des tumeurs chez les souris infectées par le virus MSV et traitées par l'AZT (figure 10) et par son dérivé 5'hémisuccinate selon l'invention (figure 11).

aux figure 10 :
o = contrôle
● = 5 mg/kg/jour d'AZT
▲ = 25 mg/kg/jour d'AZT
■ = 125 mg/kg/jour d'AZT

figure 11 :
o = contrôle
● = 6,8 mg/kg/jour de 5'hémisuccinyl-AZT
▲ = 34 mg/kg/jour de 5'hémisuccinyl-AZT
■ = 170 mg/kg/jour de 5'hémisuccinyl-AZT

**<u>EXEMPLE 1</u> : Préparation du 5' hemisuccinyl-3'azido-3'déoxythymidine**

La réaction a lieu selon le schéma suivant :

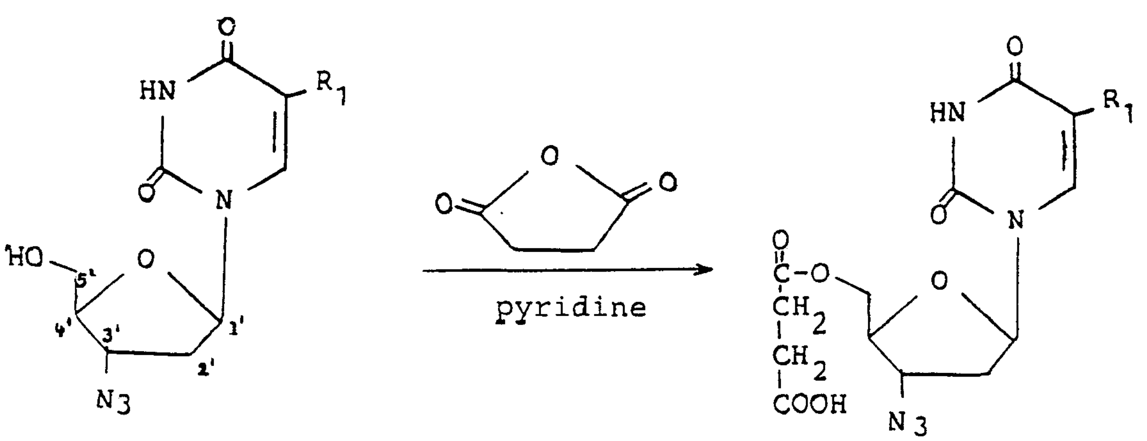


De l'anhydride succinique (207 mg) est ajouté à la température ambiante à une solution de 3'azido-3'déoxythymidine (369 mg) dans la pyridine (10 ml). Le mélange est agité à température ambiante pendant 15 heures. Après contrôle de l'avancement de la réaction par CCM (chromatographie sur couche mince) la pyridine est évaporé sous vide et le résidu est purifié par chromatographie sur gel de silice 60 en utilisant comme système d'éluants un mélange d'éther, de méthanol et d'ammoniaque (65:35:0,5). Les fractions contenant le produit désiré sont rassemblées, concentrées sous vide et lyophylisées.

Après trituration dans l'acétonitrile, on obtient 450 mg de 5'-hémisuccinyl-3'-azido-3'-déoxythymidine sous forme d'une poudre blanche (rendement : 89 %).

UV (NaCl 9°/oo) (nm) λ max (ε) 266 (9730), 209 (9901)

MS : 368 (M+1), 350, 325, 250, 242, 207, 199, 185, 125

$H^1$ RMN (DMSO-d6) 7.62 (s, 1H, H-6), 6.21 (t, 1H, H-1'), 4.57 (m, 1H, H-3'), 4.38 (m, 1H, H-5'A), 4.23 (m, 1H,

H-5'B), 4.01 (m, 1H, H-4'), 2.64 - 2.23 (m, 6H, H-2' + 2 x $CH_2$), 1.83 (s, 3H, $CH_3$)
IR : KBr : 3200, 2819, 2100, 1745, 1708, 1680, 1665, 1532, 1472, 1270, 1159, 1100 $cm^{-1}$.

**EXEMPLE 2 : Préparation du 5'-O-(N-acétyl-L-hemiaspartyl)-3'-azido-3' déoxythymidine 2**

La réaction a lieu selon le schéma suivant :

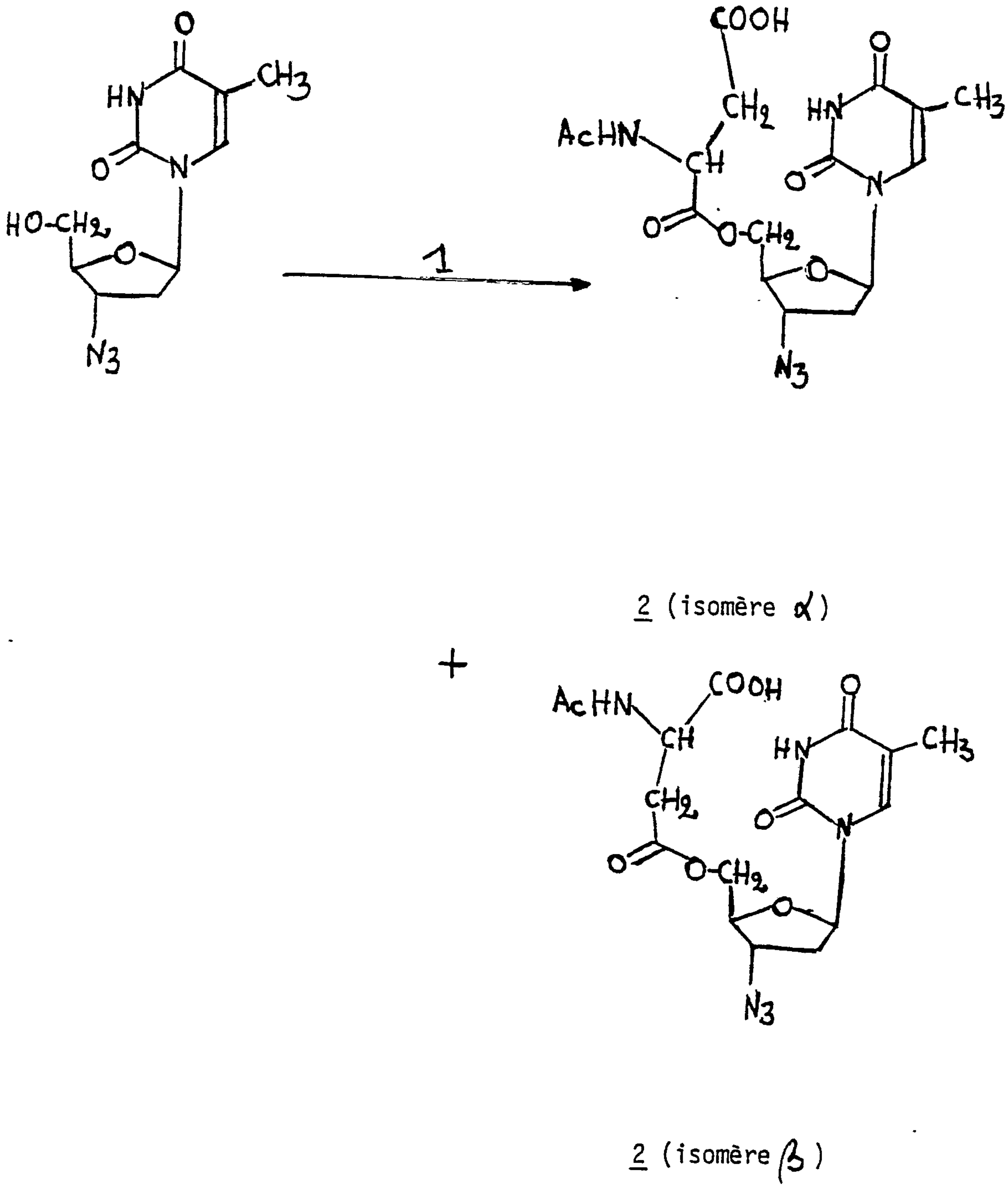


2 (isomère α)

+

2 (isomère β)

1) Anhydride N-acetyl-L-aspartique 1

Une suspension de 500 mg d'acide N-acetyl-L-aspartique dans 5 ml d'anhydride acétique est chauffée à 55°C, à l'abri de l'humidité jusqu'à l'obtention d'une solution limpide ( ± 3 heures).

L'excès d'anhydride est distillé sous vide. Le résidu blanc est trituré avec un mélange acétone-éther de pétrole. On obtient 393 mg d'anhydride N-acetyl-L-aspartique.

CARACTERISTIQUES DE 1

-$^{1}H$ RMN (DMSO-$D_6$)
1.88 (3H,s,$CH_3$),2.85 (1H,dd,Hβ),3.22 (1H,dd,Hβ), 4.61 (1H,m,Hα), 8.92 (1H,m,NH)

-$^{13}C$ RMN (DMSO -$D_6$)
21.57 ($CH_3$), 34.71 ($CH_2$), 49.40 (CH), 170.13 (CO), 170.24 (CO), 171.72 (CO).

2) 5′-O-(N-acetyl-L-hemiaspartyl)-AZT 2

Une solution d'AZT (200 mg, 0.75 mmol) et d'anhydride N-acetyl-L-aspartique 1 (160 mg, 1.02 mmole) dans la pyridine (5 ml) est agitée à 25°C pendant 24 heures.

Le solvant est évaporé sous pression réduite et le résidu est analysé par HPLC (sur une colonne μ-Bondapak C18, équilibrée par un mélange tampon phosphate (pH:6.90)-acétonitrile 9-1 à un flow de 2 ml/min).

L'analyse HPLC indique la présence des deux composés hémiaspartates dans un rapport 7:3 ($T_r$ = 5.60 et 6.60 min).

Le mélange d'isomères est séparé par chromatographie sur silica gel en éluant avec un mélange acétate d'éthyle-acétone-$H_2O$ 5-10-1 contenant 0.5 % de pyridine.

On obtient 220 mg du dérivé hémiaspartyl 2 (isomère majeur : $T_r$ = 5.60 min) sous forme d'une poudre blanche après trituration dans l'acétonitrile.

CARACTERISTIQUES DE 2

-$^{1}H$ RMN (DMSO-$D_6$)
8.24 (1H,m,NH),7.51 (1H,s,H-6), 6.13(1H,m,H-1′), 4.67-4.40(2H,m,H-3′ + CH*aa), 4.37-4.15 (2H,m,H-5′), 3.97 (1H,m,H-4′), 2.57-2.37 (3H,m,$CH_2$+H-2′B), 2.35-2.22 (1H,m,H-2′A), 1.82 (3H,s,$CH_3$),1.80(3H,s,$CH_3$)

- UV($CH_3OH$) : λ(ε)
264(7.546), 232 (2.214), 202.6

- IR (KBr)
3409,2110,1702,1412,1271,1099,562 $cm^{-1}$

MASSE
-FAB (+) : 425 (M+1), 399, 277, 256, 223, 207, 185, 153, 127, 115, 93.
-FAB (-) : 423 (M-1), 397, 266, 205, 183, 156, 125, 91

On obtient 60 mg du dérivé hémiaspartyl 2 (isomère mineur : $T_r$ = 6.60 min) après trituration dans l'acétonitrile. Rendement total : 80 % (2 isomères).

CARACTERISTIQUES DE 2 (MINEUR)

-$^{1}H$ RMN (DMSO-$D_6$)
7.67 (1H,m,NH), 7.57 (1H,s,H-6), 6.14 (1H,m,H-1′), 4.60 (1H,m,H-3′),4.42-4.04 (3H,m,H-5′ + CH*aa), 3.94 (1H,m,H-4′), 2.80-2.21 (4H,m,$CH_2$ + H-2′), 1.81 (3H,s,$CH_3$), 1.79(3H,s,$CH_3$)

-MASSE
FAB (+) : 425 (M+1), 277, 256, 223, 185

-IR (KBr)
3854, 3751, 3676, 3650, 3392, 2210, 1685, 1419, 1273, 1095, 558 $cm^{-1}$

**EXEMPLE 3 : Préparation du 5′-O-hémiglutaryl-3′-azido-3′-déoxythymidine 3**

La réaction se fait selon le schéma suivant :

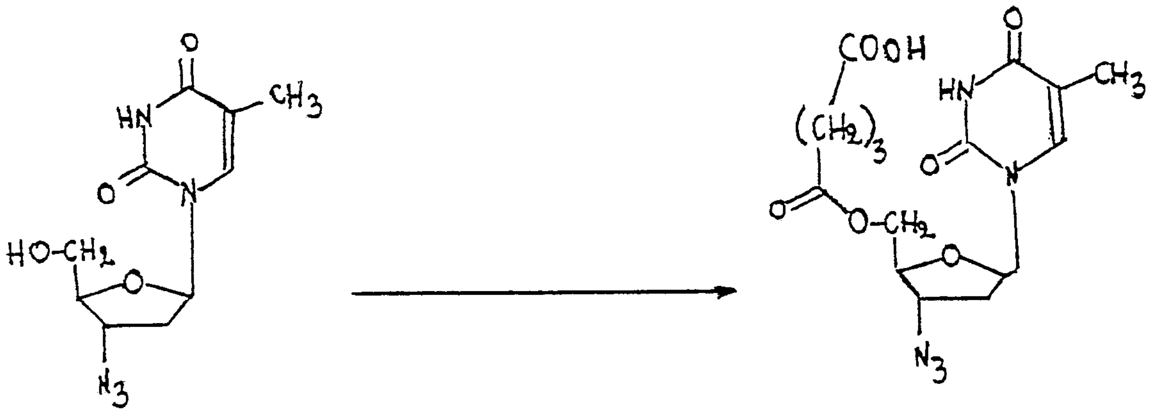

3

5'-O-HEMIGLUTARYL-3'-AZIDO-3'-DEOXYTHYMIDINE 3

De l'anhydride glutarique (137 mg, 1.2 mmole) est ajouté à une solution d'AZT (200 mg, 0.75 mmole) dans la pyridine (5ml).

Le mélange est agité à température ambiante à l'abri de l'humidité pendant 24 heures.

Après évaporation du solvant sous vide, le résidu est chromatographié sur une colonne de silica-gel équilibrée avec un mélange ether-métanol-$NH_4OH$ aq. 60-39.5-0.5

On obtient après trituration dans l'acétonitrile 280 mg du dérivé hemiglutaryl 3 sous forme d'une poudre blanche.

Rendement 61 %.

CARACTERISTIQUES DE 3

-$^1H$ RMN (DMSO-$D_6$)
7.45 (1H,S,H-6),6.12 (1H,m,H-1'), 4.47(1H,m,H-3'), 4.25 (2H,m,H-5') 3.97 (1H,m,H-4'),2.56-2.26 (4H,m,H-2'+$CH_2$), 2.10 (2H,m,$CH_2$), 1.79 (3H,s,$CH_3$),1.71(2H,m,$CH_2$)

- $^{13}C$ RMN (DMSO-$D_6$)
175.6, 172.5, 163.6, 150.3, 135.8, 109.9, 85.58, 80.61, 63.1, 60.1, 35.6, 34.7, 32.9, 20.6, 12.

- IR (KBr)
3750,3676,3426,2927,2109,1702, 1652,1410,1268,1099 $cm^{-1}$

MASSE
-FAB (+) : 404 (M+Na), 382(M+H), 356, 277, 223, 207, 185, 153, 131, 127, 115, 93.
-FAB (-) : 402(M+Na-H), 380 (M-H), 354, 329, 297, 275, 266, 255, 237, 223, 205, 183, 145, 125, 113, 91

**EXEMPLE 4 : Préparation du 5'-O-sulfobenzoyl-3'-azido-3'-déoxythymidine 4**

Le schéma réactionnel est le suivant :

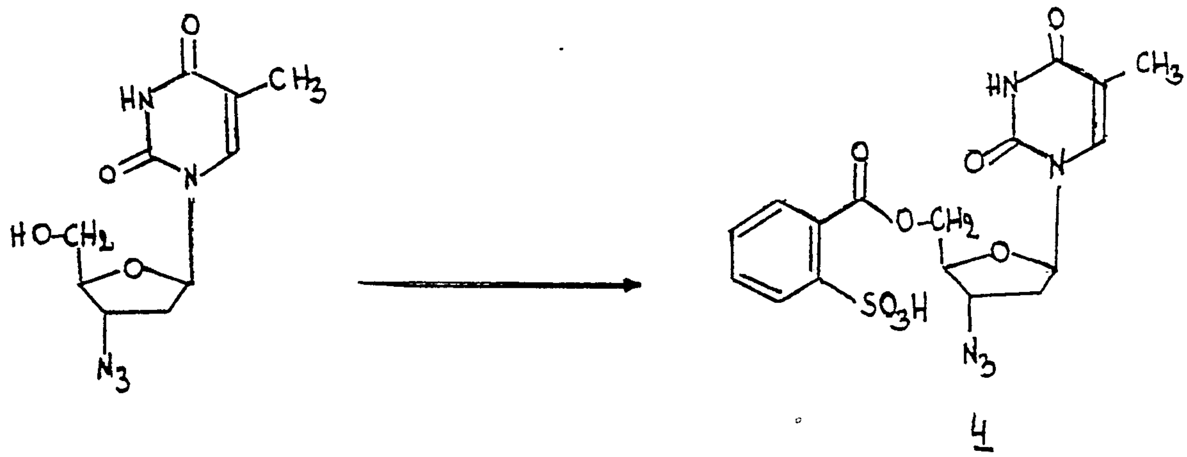

4

5′-O-SULFOBENZOYL-3′-AZIDO-3′-DEOXYTHYMIDINE 4

Une solution d'AZT (200 mg, 0.75 mmole) et d'anhydride cyclique 2-sulfobenzoïque (221 mg, 1.2 mmole) dans la pyridine (5ml) est agitée à température ambiante et à l'abri de l'humidité pendant 24 heures. Un traitement similaire à celui décrit pour le dérivé 3 permet d'obtenir 281 mg du dérivé sulfobenzoyl après trituration dans un mélange méthanol-éther.

Rendement 83 %.

CARACTERISTIQUES DE 4

MASSE
-FAB (-) : 450.1 (M-1), 424, 407, 329, 298, 280, 215, 201, 179, 157, 125
-FAB (-) : Daughters of 450.1 450.1, 422.0, 407.0, 296.5, 296.1, 266.0, 201, 157, 125

- IR (KBr)
3202, 2110, 1699, 1439, 1192, 1081, 1018, 759, 615, 564 $cm^{-1}$

-$^1H$ RMN (DMSO-$D_6$)
8.07-7.29 (5H,m,aromatiques + H-6),6.26(1H,m,H-1′), 5.29(1H,m,H-3′), 4.48 (1H,m,H-5′A), 4.33 (1H,m,H-5′B), 4.07 (1H,m,H-4′),2.48 (1H,m,H-2′A), 2.33 (1H,m,H-2′B), 1.26(3H,s,$CH_3$)

**EXEMPLE 5 : Préparation des 5′-O-(Boc-L-yalyl) déoxythymidine 6 et 5′-O-(L-valyl)-3′-azido-3′-déoxythymidine 7**

Le schéma réactionnel est le suivant :

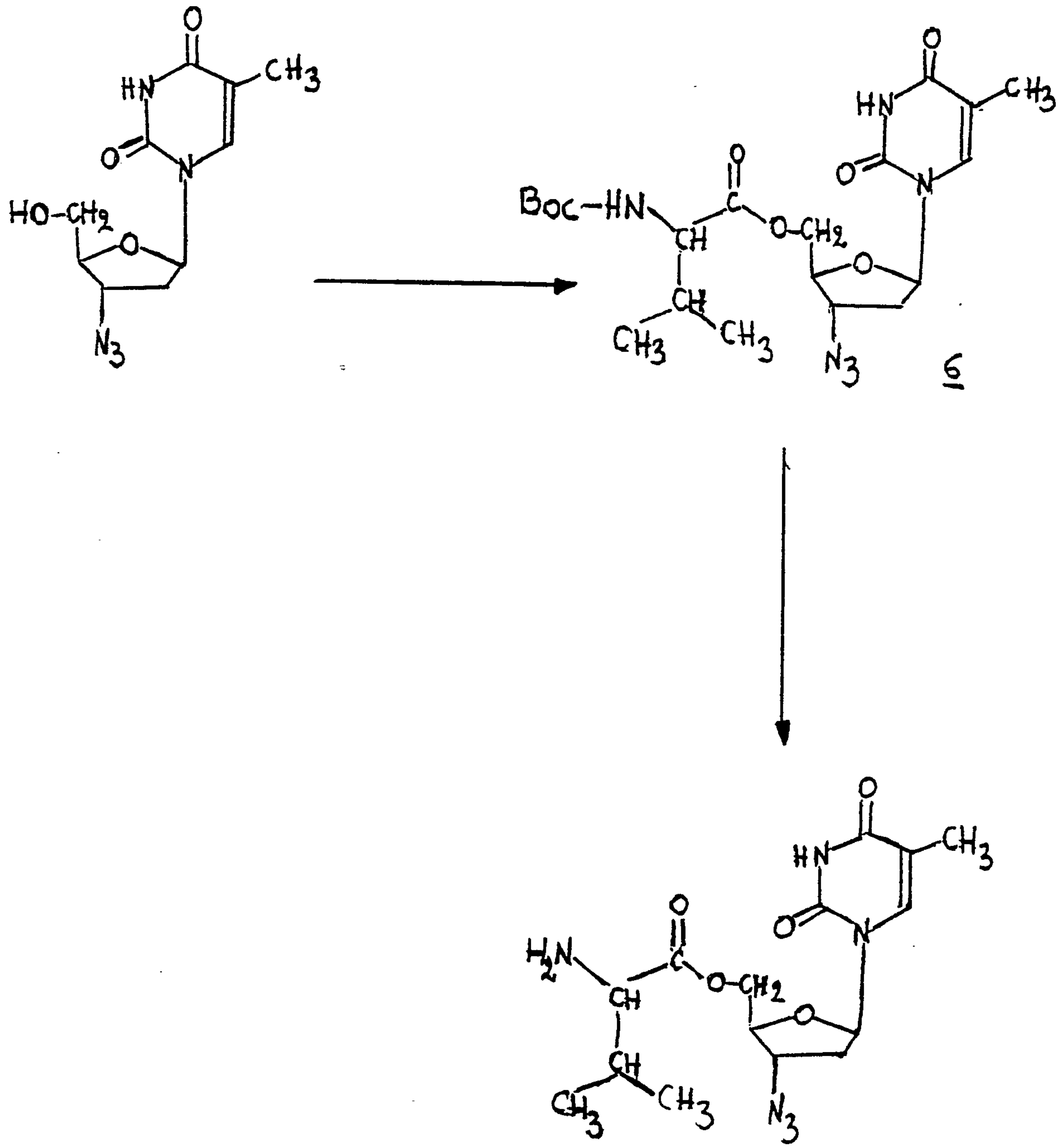

6

7

5′-O-(Boc-L-VALYL)-3′-AZIDO-3′-DEOXYTHYMIDINE 6

Une solution de 3′-azido-3′-deoxythymidine (150 mg, 0.56 mmole) et du p-nitrophenylester de la N-Boc-L-valine (397 mg, 1.12 mmole) dans la DMF (3 ml) est agitée 20 heures à température ambiante en présence de DBU (168µl). La DMF est évaporée sous pression réduite. Le résidu est dissout dans de l'acétate d'éthyle (30 ml). La solution organique est lavée successivement par une solution aq. en $Na_2CO_3$(1M), par de l'eau et par une solution aqueuse saturée en NaCl. La phase organique est séchée, concentrée sous vide et est purifiée par chromatographie sur silica gel (éluant : acétate d'éthyle).

On obtient de cette façon la 5′-O-(Boc-L-valyl)-3′-azido-3′-deoxythymidine 6 sous forme d'une poudre blanche après trituration dans l'acétonitrile.

Rendement 71 % (185 mg)

CARACTERISTIQUES DE 6

MASSE
-FAB (-) : 465 (M-1), 450, 391, 348, 270, 249,216 183, 151, 142, 125, 99, 91. 71.
-FAB (-) : DAUGHTERS OF 465 465, 422, 391, 348, 223,142, 125

- IR (KBr)
3854, 3751, 3676, 3650, 3397, 2973, 2110, 1700, 1560, 1508, 1474, 1367, 1273, 1159, 1088 $cm^{-1}$

-$^1H$ RMN ($DMSO-D_6$)
11.38 (1H,s,NH),7.44 (1H,m,H-6), 6.13(1H,m,H-1′), 4.44 (1H,m,CH*aa), 4.28(2H,m,H-5′),4.00(1H,m,H-3′), 3.86(1H,m,H-4′), 2.43(1H,m,H-2′A), 2.32 (1H,m,H-2′B), 2.01 (1H,m,CH), 1.80 (3H,s,$CH_3$),1.38(9H,s,$C(CH_3)_3$), 0.876(6H,m,2$CH_3$)

- $^{13}C$ RMN ($DMSO-D_6$)
171.7, 163.5, 155.6, 150.2, 135.7, 109.8, 83.5, 80.3, 78.1, 63.7, 60.2, 59.4, 35.3, 29.4, 28.0, 18.8, 18.3, 11.9.

5′-O-(L-VALYL)-3′-AZIDO-3′-DEOXYTHYMIDINE 7

Une solution de 5′-O-(Boc-L-valyl)-3′-azido-3′-deoxythymidine 6 (440 mg, 0.94 mmole) dans un mélange (8ml) de dichloromethane-acide trifluoroacétique (5-3) est agitée une heure à température ambiante à l'abri de l'humidité. Les solvants sont évaporés sous pression réduite. Le résidu est dissout dans 35 ml d'eau et extrait par 2 x 20 ml d'acétate d'éthyle. Ces phases organiques sont éliminées. La solution aqueuse est ensuite amenée à pH basique par addition de $NH_4OH$aq. et est extraite par 3 x 20 ml d'acétate d'éthyle. Les phases organiques sont rassemblées, lavées par une solution aqueuse saturée en NaCl, séchée sur $MgSO_4$ et concentrée. Le résidu obtenu est purifié par chromatographie sur silica gel (élution : acétate d'éthyleméthanol 9-1).

Les fractions 14-20 permettent d'obtenir 253 mg de 5′-O-(L-valyl)-3′-azido-3′-deoxythymidine 7.
Rendement 73 %.

CARACTERISTIQUES DE 7

-$^1H$ RMN ($DMSO-D_6$)
7.52 (1H,s,H-6),6.18 (1H,m,H-1′), 4.55(1H,m), 4.37 (2H,m,H-5′) 4.04 (1H,m), 3.22 (1H,m), 2.48 (1H,m,H-2′A), 2.37 (1H,m,H-2′B), 1.88 (1H,m,CH) 1.83(3H,s,$CH_3$), 0.91(6H,m, 2$CH_3$)

- $^{13}C$ RMN ($DMSO-D_6$)
174.8, 163.6, 150.3, 135.9, 109.8, 83.4, 80.4, 63.1, 60.0, 59.5, 35.4, 31.7, 19.1, 17.3, 12.0

**EXEMPLE 6 : Préparation du 5′-O-(L-pyroglutamyl)-3′-azido-3′-déoxythymidine 9**

Le schéma réactionnel est le suivant :

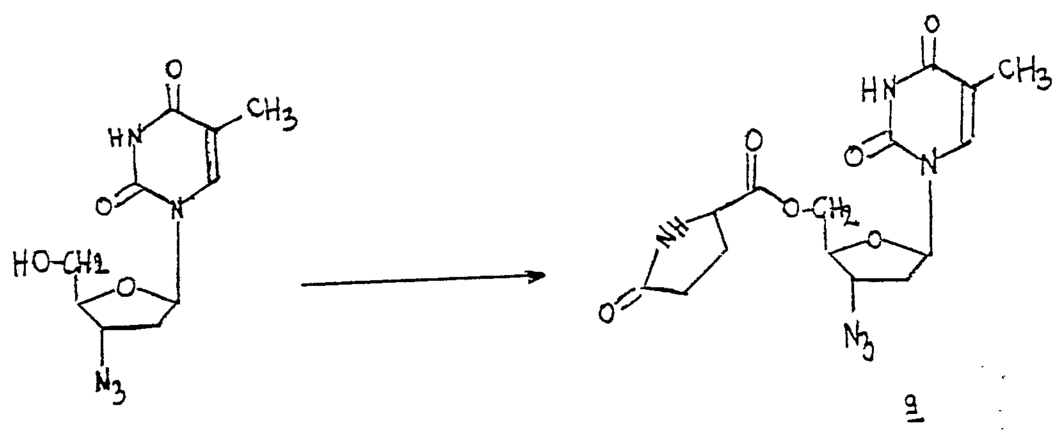

9

5′-O-(L-PYROGLUTAMYL)-3′-AZIDO-3′-DEOXYTHYMIDINE 9

De l'acide L-pyroglutamique (188 mg, 1.45 mmole) est dissout dans la DMF (15 ml) en présence de N-methylmorpholine (161 µl).

Du N,N′-disuccinimidylcarbonate (374 mg, 1.46 mmole) est ajouté et la solution est agitée 4 heures à

température ambiante, à l'abri de l'humidité.

Une solution de 3'-azido-3'-deoxythymidine (300 mg, 1.12 mmole) dans la DMF (9ml) est ensuite ajoutée ainsi que du 1,8-diazabicyclo (5.4.0) undec-7-ene (218 µl).

Après 20 heures d'agitation, la solution est concentrée sous pression réduite. Le résidu obtenu est purifié par chromatographie sur silica gel (éluant : acetate d'éthyle-méthanol 97-3) et par cristallisation dans l'acétonitrile.

Il permet d'obtenir 328 mg de 5'-O-(L-pyroglutamyl)-3-azido-3'-deoxythymidine.

Rendement 59.8 %.

CARACTERISTIQUES DE 9

-$^{1}$H RMN (DMSO-$D_6$)

11.36(1H,s,NH), 8.02(1H,s,NH), 7.40 (1H,s,H-6), 6.11(1H,m,H-1'), 4.58 (1H,m,H-3'), 4.33 (2H,m,H-5'), 4.23(1H,m,CH * aa), 3.99 (1H,m,H-4'), 2.61 - 1.97(6H,m,H-2' + $2CH_2$), 1.80 (3H,s,$CH_3$)

-IR (KBr)

3854, 3751, 3676, 3649, 3421, 2108, 1700, 1508, 1458, 1272 $cm^{-1}$

**EXEMPLE 7 : Préparation du 5'O-L-leucyl-3'azido-3'déoxythymidine**

La réaction a lieu selon le schéma suivant :

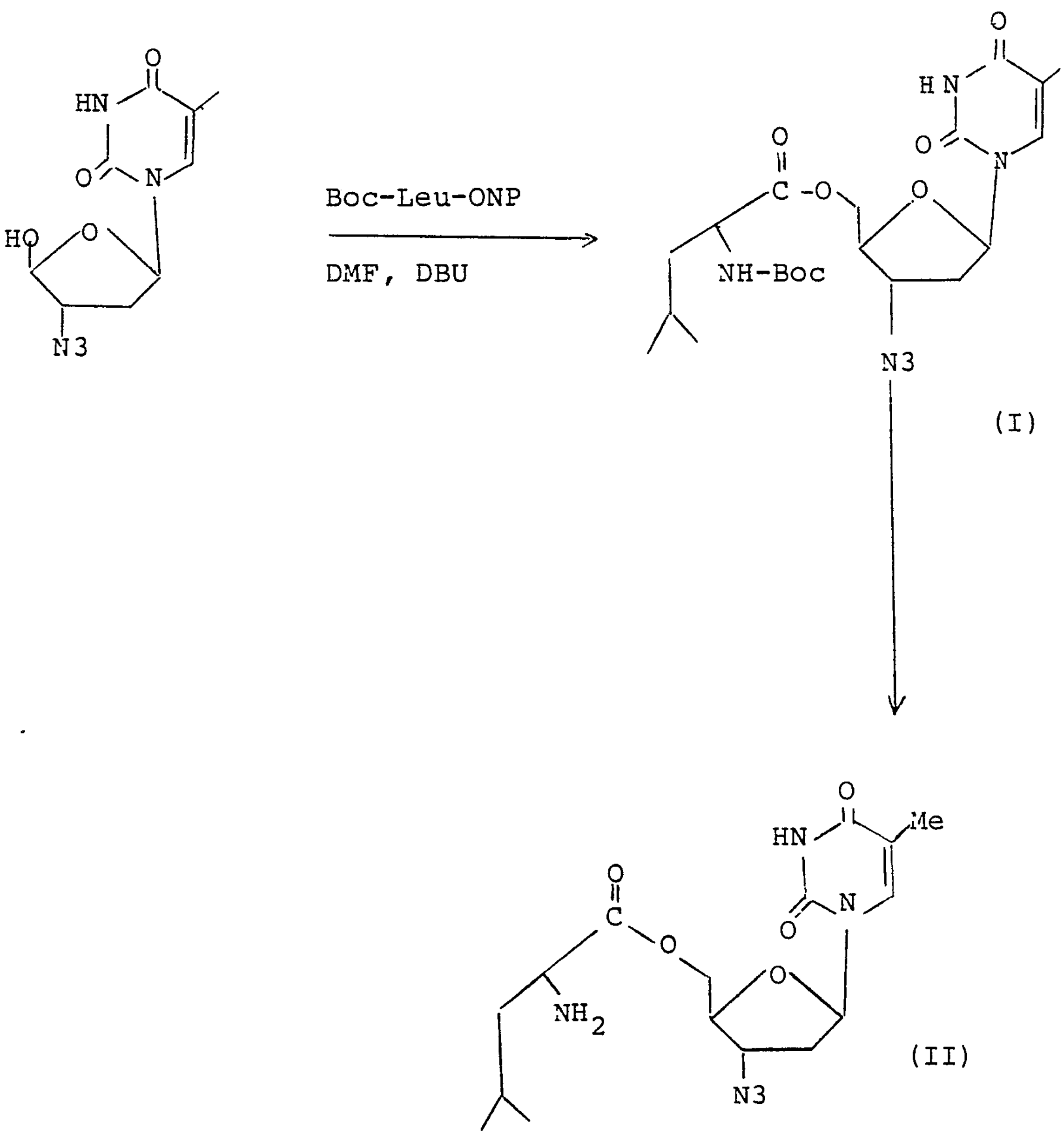


(I)

(II)

5'O-(Boc-L-leucyl)-3'-azido-3'-déoxythymidine (I)

A une solution de 3'azido-3'-déoxythymidine (150 mg, 0.561 mmoles) et du p-nitrophenyl ester de la N-Boc-L-leucine (395 mg, 1.122 mmoles) dans la N, N-dimethylformamide (3 ml), on ajoute à température ambiante et sous agitation du 1,8-diazabicyclo [5.4.0] undec-7-enc (168 µl, 1.123 mmoles).

Après 15 h à température ambiante, l'analyse par CCM (acétate d'éthyle) indique la transformation complète du produit initial.

Le mélange réactionnel est concentré sous pression réduite. Le résidu est dissout dans l'acétate d'éthyle (40 ml), lavé par une solution aqueuse de $Na_2CO_3$ (1M, 2 X 20 ml), par de l'eau (20 ml) et par une solution aqueuse saturée en NaCl (20 ml). La phase organique est séchée à l'aide de $MgSO_4$ et est concentrée jusqu'à l'obtention d'une huile jaune qui est purifiée par chromatographie sur silice (40 gr de Kieselgel 60/0.04-0.063 mm) éluant : acétate d'éthyle). Le produit désiré est obtenu sous forme d'une poudre blanche après évaporation des fractions 9 à 13 et trituration dans un mélange acétate d'éthyle-éther de pétrole.

Rdt : (201 mg, 74.6 %)

Masse : FAB - 479.2 (M-1), 453.2, 406.2, 405.2, 379.2, 362.1, 310.1, 230.1, 201.9, 157.0, 156.0

FAB- (daughters of 479.2), 479.2, 436.2, 405.2, 362.1, 266.2, 223, 212.1, 156

IR KBr ($cm^{-1}$) : 3360, 3190, 2960, 2935, 2875, 2110, 1745, 1700, 1518, 1470, 1369, 1273, 1165, 1102, 1052

RMN ($CDCl_3$, 300 MHz) : 8,71 (NH, 1H), 7.17 (s, 1H), 6.06 (m, 1H), 4.92 (m, 1H), 4.45 - 4.15 (m, 3H); 4.08 (m, 1H), 2.55 - 2.28 (m, 2H), 1.95 (s, 3H), 1.84 -1.49 (m, 3H), 1.43 (s, 9H), 0.96 (d, 6H)

5'O-(L-leucyl)-3'-azido-3'déoxythymidine (II)

Une solution de 5'O-(Boc-L-leucyl)-3'azido-3'déoxythymidine (160 mg, 0.333 mmoles) dans l'acide trifluoroacétique (3 ml) est agitée à température ambiante pendant 4 heures.

Le mélange réactionnel est concentré sous pression réduite et le résidu est purifié par chromatographie sur silice (40 g de Kieselgel 60, éluant : acétate d'éthyle/méthanol : 95/5). Le produit désiré est obtenu sous forme de mousse après évaporation des fractions 20 - 37.

Rdt : (112 mg, 88 %)

Masse : EI + : 381 (M+1), 355, 338, 289, 277, 259, 255, 227, 185, 180 EI - : 379 (M-1), 297, 275, 255, 227, 205, 183 EI + : (daughters of 381) : 381, 255, 250, 227, 212

RMN ($CDCl_3$ + DMSO, 300 MHz) : 7.20 (s, H-6), 6.06 (m, H-1'), 4.35 (m, 2H), 4.30 (m, 1H) 4.06 (m, 1H), 3.55 (m, 1H), 2.44 (m, 2H-2'), 1.91 (s, Me), 1.49 (m, 2H), 1.78 (m, 1H), 0,94 (d, 2 Me)

IR (KBr $cm^{-1}$) : 3200, 2960, 2110, 1735, 1690, 1470, 1275, 1205

**EXEMPLE 8 : stabilité in vitro des dérivés de l'AZT selon l'invention**

La stabilité des dérivés de l'AZT, et plus particulièrement des dérivés hémissuccinate et hémiaspartate, selon l'invention, a été testée dans différentes conditions.

1) Stabilité des dérivés de l'AZT en fonction du pH

On prépare des solutions du dérivé de l'AZT (200 µg/ml) et d'un standard interne (200 µg/ml) dans des tampons glycine-HCl (40 mM) de différents pH (respectivement aux pH 1,02-2,9-8,9-11,07).

Les solutions sont agitées à température ambiante. Des aliquotes (25 µl) sont prélevées à différents moments (0,1 h, 2 h, 4 h, 6 h, 24 h, 48 h), diluées par 475 µl de tampon phosphate (0,02 M, pH 6,82) et analysées par HPLC sur une colonne µBondapak C18 équilibrée avec un mélange tampon phosphate (20 mM pH 6,82) et acétonitrile 10 % à un débit de 2 ml/min.

Les résultats pour le dérivé 5'hémisuccinyl et 5'hémiaspartyl de l'AZT sont présentés dans les figures 1 à 3.

2) Stabilité des dérivés de l'AZT en présence d'enzymes lysosomiales

On prépare une solution du dérivé de l'AZT (120 µg/ml) et d'un standard interne (120 µg/ml) dans du tampon acétate (40 mM pH 4,5 ou 5,5) contenant de la cystéine (5 mM) ainsi que des enzymes lysosomiales 1,2 mg/ml). La solution initiale est préparée au départ des différentes solutions stériles décrites ci-dessous. Les solutions de cystéine et de TS sont ajoutées en dernier lieu.

| | Concentration finale | Volume |
|---|---|---|
| Tampon acétate 1M | 40 mM | 12 µl |
| Cystéine 0,1 M ($H_2O$) | 5 mM | 15 µl |
| TS 3,41 mg/ml | 1,2 mg/ml | 106 µl |
| Produit 1 mg/ml ($H_2O$) | 120 µg/ml | 36 µl |
| Standard Interne 1 mg/ml ($H_2O$) | 120 µg/ml | 36 µl |
| $H_2O$ | | 93 µl |
| | | 300 µl |

Les résultats pour le dérivé 5'hémisuccinyl de l'AZT sont présentés dans la figure 4.

Le mélange est incubé à 37° C. Des aliquotes sont prélevées à différents moments (0,1 h, 2 h, 4 h, 6 h, 24 h, 48 h), traitées par deux volumes de méthanol (600 µl), et microfugées pendant 5 minutes à 13000 RPM.

Une partie du surnageant est analysée par HPLC sur une colonne Bondapak C18 équilibrée avec un mélange tampon phosphate (20 mM pH 6,82) 90 % et acétonitrile 10 % à un débit de 2 ml/min.

3) Stabilité des dérivés de l'AZT en présence de sérum et de plasma

On prépare une solution du dérivé de l'AZT (120 µg/ml) et d'un standard interne (120 µg/ml) dans un mélange de sérum (ou de plasma humain) et de PBS.

La solution initiale est préparée au départ des différentes solutions stériles décrites ci-dessous.

| | Concentration finale | Volume |
|---|---|---|
| Sérum (ou plasma) | | 1 400 µl |
| PBS 5X | PBSIX | 500 µl |
| Produit 1 mg/ml ($H_2O$) | 120 µg/ml | 300 µl |
| Standard interne 1 mg/ml ($H_2O$) | 120 µg/ml | 300 µl |
| | | 2 500 µl |

Le mélange est incubé à 37° C. Des aliquotes sont prélevées à différents moments (0,1 h, 2 h, 4 h, 6 h, 24 h, 48 h), traitées par deux volumes de méthanol (600 µl) et microfugées pendant 5 minutes à 13000 RPM.

Une partie du surnageant est analysée par HPLC sur une colonne µBondapak C18 équilibrée avec un mélange tampon phosphate (20 mM pH 6,82) 90 % et acétonitrile 10 % à un débit de 2 ml/min.

Les résultats sont présentés dans les figures 5 à 7 pour les dérivés 5'hémisuccinyl et 5'hémiaspartyl de l'AZT.

**EXEMPLE 9 : activité in vitro des dérivés de l'AZT selon l'invention**

Des expériences pour établir l'activité anti-HIV des dérivés des exemples 1 et 2 sur :

1. cellules MT4 infectées par le virus HIV ; et
2. cellules HUT/78 infectées par le virus HIV.

1. Expériences sur cellules MT4 infectées par le virus HIV

Les produits (AZT, AZT-5' hémisuccinyl et AZT 5'-leucyl) à différentes concentrations ont été mis en présence de cellules MT4 ($4 \times 10^4$ cellules par puits de microtray) infectées ou non avec 100 $CCID_{50}$ de virus HIV-1 (souche HTLV-$III_B$). La $CCID_{50}$ représente la quantité de virus à rajouter pour obtenir une infection de 50 % des cellules (dose infectieuse de cellules en culture 50).

Après cinq jours d'incubation à 37° C, le nombre de cellules vivantes a été déterminé par la méthode au MTT, basée sur la réduction par les cellules vivantes du 3'-(4, 5-diméthylthiazol-2-yl)-2,5-diphényltétrazoliumbromide, jaune, en formazan, bleu. L'activité antivirale et la cytotoxicité sont exprimées sous forme de $ED_{50}$ et de $CD_{50}$. La $CD_{50}$ représente la dose qui tue 50 % des cellules non infectées et la $ED_{50}$ représente la dose qui protège 50 % des cellules de l'infection virale.

Les résultats sont exprimés au tableau 2 en $CD_{50}$, $ED_{50}$ et SI qui représente l'indice de sélectivité et qui est égal à $CD_{50}$ sur $ED_{50}$.

Tableau 2

activité in vitro des dérivés de l'AZT selon l'invention

| Exp.n° | Produit | $CD_{50}$ | $ED_{50}$ | SI | SI dérivé/SI AZT |
|---|---|---|---|---|---|
| 1 | AZT<br>AZT 5'hémisuccinyl | 15.8 µM<br>196 µM | .0026 µM<br>.0069 µM | 6101<br>28543 | 4.7 |
| 2 | AZT<br>AZT-5'leucyl | 14.2 µM<br>25.4 µM | .006 µM<br>.005 µM | 2372<br>5080 | 2.1 |

2. Expériences sur cellules HUT-78 infectées par le virus HIV

Les produits (AZT et AZT-5' hémisuccinyl) à différentes concentrations ont été mis en présence de cellules HUT-78 infectées par le virus HIV-1 (souche HTLV-III$_B$) à une multiplicité d'infection de 0.4. Tous les quatre jours, 3/4 du milieu ont été remplacés par du milieu frais. Après douze jours, l'expression des antigènes viraux a été mesurée par immunofluorescence indirecte à l'aide d'un sérum anti-HIV-I positif, et par cytofluorescence laser, tel que décrit par Pauwels et al. J. Virol. Meth. 16, 171-185 (1987).

Des résultats sont exprimés dans le tableau 3 en $ED_{50}$ et en pourcentage d'effet protecteur selon la formule :

$$100 \times 1 - \frac{\text{nbre cellules positives en présence du produit - nbre cellules contrôles positives}}{\text{nbre cellules positives en absence du produit - nbre cellules contrôles positives}}$$

TABLEAU 3

| Concentration (µM) du produit | AZT | Pourcentage d'effet protecteur AZT-5'hémisuccinyl |
|---|---|---|
| 0 | 0 | 0 |
| 0.0004 | | - 74 |
| 0.0008 | - 41 | |
| 0.022 | | -23 |
| 0.04 | - 19 | |
| 0.11 | | 48 |
| 0.2 | 36 | |
| 0.54 | | 21 |
| 1 | 45 | |
| 2.71 | | 72 |
| 5 | 65 | |

$ED_{50}$ AZT = 2 µM

$ED_{50}$ AZT-5'hémisuccinyl = 1,77 µM

3) Résultats supplémentaires concernant les dérivés de l'AZT

3.1) Activité in vitro du dérivé 5′hémisuccinyl

3.1.1) Expériences sur cellules MT-4 infectées par le virus HIV

La même expérience que celle décrite ci-avant (exemple 9.1)) a été réalisée, mais en présence de thymidine 250 µM et de 2′déoxycytidine 500 µM. En effet l'AZT entre en compétition avec la thymidine au niveau des enzymes de phosphorylation qui vont le transformer en AZT triphosphate (AZT TP) qui est la forme active du produit. Un excès de thymidine réduit donc très fortement l'activité de l'AZT, ce qui se traduit par une $ED_{50}$ plus élevée. Par conséquent, si le dérivé de l'AZT doit son activité à la libération d'AZT qui à son tour est phosphorylé en AZT TP, un excès de thymidine aura également pour effet d'élever la $ED_{50}$ du dérivé. La thymidine étant cytotoxique à la concentration utilisée, il est nécessaire de rajouter de la 2′déoxycytidine pour empêcher cette toxicité.

Les résultats sont repris au tableau 4

Tableau 4

Activité in vitro de l'AZT et de l'AZT-5′hémisuccinyl sur cellules MT-4 infectées par le virus HIV-1 en absence et présence de thymidine

| Produit | thymidine (250µM) + 2′déoxy-cytidine (500 µM) | $CD_{50}$ (µM) | $ED_{50}$ (µM) | SI |
|---|---|---|---|---|
| AZT | - | 5.06 | 0,0018 | 2780 |
| AZT 5′ hémisuccinyl | - | 295 | 0,0523 | 5633 |
| | + | 362 | 6,8399 | 53 |

3.1.2) Expériences sur lymphocytes de sang périphérique humain infectés par le virus HIV

Les lymphocytes ont été extraits d'un concentré plaquettaire par séparation sur gradient de Ficoll et élimination des adhérentes. Après 3 jours de stimulation par la phytohémaglutinine, ils ont été transférés dans un milieu additionné d'interleukine-2 (IL-2) est infectés par le virus HIV-1 (souche HTLV-IIIB). L'AZT et son dérivé 5′ hémisuccinate à différentes concentrations ont été rajoutés aux cellules au moment de l'infection.

Trois jours après l'infection, le milieu a été remplacé par du milieu frais additionné d'IL-2, contenant les produits aux concentrations désirées pour la moitié des cellules, et ne contenant plus de produit pour l'autre moitié. Ensuite, le milieu des cellules a été remplacé à moitié par du milieu frais additionné d'IL-2, contenant ou non les produits aux concentrations désirées, tous les 3 à 4 jours.

La multiplication du virus a été suivie par dosage des antigènes viraux dans les surnageants de culture (Innotest HIV-Organon Teknika). Les résultats exprimés en $ED_{50}$ au jour 14 de culture, sont repris au tableau 5.

Tableau 5

Activité in vitro de l'AZT et de l'AZT 5′hémisuccinyl sur lymphocytes de sang périphérique humain infectés par le virus HIV-1 (jour 14)

| Produit | Durée d'administration (Jours) | $ED_{50}$ (µM) |
|---|---|---|
| AZT | 3 | 0.03 |
| | 14 | 0.023 |
| AZT 5′hémisuccinyl | 3 | 0.078 |
| | 14 | 0.057 |

3.2) Activité in vitro d'autres dérivés sur cellules MT-4 infectées par le virus HIV

Les protocoles expérimentaux sont ceux décrits à l'exemple 9 3.1) et 9 3).

Les résultats sont repris au tableau 6.

Tableau 6

Activité in vitro de l'AZT et des dérivés selon l'invention sur cellules MT-4 infectées par le virus HIV-1 en absence et présence de thymidine

| Produit | thymidine(250 μM) + déoxy-cytidine (500 μM) | $CD_{50}$ (μM) | $ED_{50}$ (μM) | SI |
|---|---|---|---|---|
| AZT | - | 5,06 | 0.0018 | 2780 |
| AZT 5'tBoc valyl | - | 420 | 0.0731 | 5740 |
| | + | 358 | 11.002 | 33 |
| AZT 5'valyl | - | 3.65 | 0.0007 | 5083 |
| | + | 38.16 | 0.0987 | 386 |
| AZT-5'hémiaspartyl (isomère majeur) | - | 65.06 | 0.0161 | 4046 |
| | + | 74.83 | 4.000 | 19 |

- AZT-5'tBoc valine : le comportement de ce dérivé se rapproche de celui de l'hémisuccinyl.- AZT-5'valine : ce dérivé montre une activité deux foix plus élevée que celle de l'AZT, mais par contre une toxicité comparable à celle de ce dernier.
- AZT-5'hémiaspartyl (isomère majeur) : ce dérivé a un indice de sélectivité comparable à celui du dérivé hémisuccinyl

**EXEMPLE 10 : activité in vivo des dérivés de l'AZT selon l'invention**

L'AZT et ses dérivés selon l'invention ont été testés in vivo chez la souris NMRI (souriceaux de 2 jours) infectée par le virus MSV Moloney sarcoma virus). La drogue est adminitrée i.p. pendant 5 jours dans un volume de 25 μl/g de poids corporel. Le virus est inoculé dans la cuisse gauche de l'animal deux heures après la première administration de drogue. Les animaux sont contrôlés tous les jours selon trois critères : survie, apparition de la tumeur et poids (toxicité) pendant 50 jours. Les résultats sont repris au tableau 7 et dans les figures 8 à 11.

% ILS = pourcentage d'augmentation du temps de survie

$$= \frac{\text{temps médian de survie}_T - \text{temps médian de survie}_C}{\text{temps médian de survie}_C} \times 100$$

On entend par temps médian de survie le temps où la moitié des souris sont encore vivantes.

% ITL = pourcentage d'augmentation du temps d'apparition de tumeur

$$= \frac{\text{temps médian d'apparition de la tumeur}_T - \text{temps médian d'apparition de la tumeur}_C}{\text{temps médian d'apparition de la tumeur}_C} \times 100$$

T = traité,

C = contrôle.

Tableau 7

activité in vivo des dérivés de l'AZT selon l'invention

| Produit et dose | Temps médian de survie | Temps moyen de survie | % ILS | Temps médian d'apparition de la tumeur | Temps moyen d'apparition de la tumeur | % ITL |
|---|---|---|---|---|---|---|
| Contrôle | 9.2 jours | 8.7 jours | - | 4.5 jours | 4.2 jours | - |
| AZT: | | | | | | |
| 5 mg/kg/jour | 11.6 jours | 11.3 jours | 26.8 | 7.3 jours | 6.5 jours | 62.8 |
| 25 mg/kg/jour | 16 jours | >19.8 jours | 74.8 | 13 jours | * | 190 |
| 125 mg/kg/jour | 16 jours | >23.3 jours | 74.8 | 10.6 jours | 10.2 jours | 137 |
| AZT-5' hémisuccinyl | | | | | | |
| 6.8 mg/kg/jour | 11.0 jours | 11.5 jours | 20.2 | 6.8 jours | 7.3 jours | 52.4 |
| 34 mg/kg/jour | >50 jours | >41 jours | >446 | 11 jours | >25.5 jours | 145 |
| 170 mg/kg/jour | 13 jours | >22.7 jours | 42.1 | 16 jours | >23 jours | 257 |

* n'est pas évaluable, plusieurs souris étant mortes avant de développer une tumeur.

**EXEMPLE 11 : Toxicité du dérivé 5'hémisuccinyl de l'AZT**

1) Toxicité in vitro

1.1) Toxicité sur lignées cellulaires continues

Dans le but d'évaluer la toxicité du dérivé 5'hémisuccinyl de l'AZT, des expériences ont été réalisées sur différentes lignées cellulaires continues.

L'AZT et son dérivé 5'hémisuccinyl à différentes concentrations ont été mis en présence de cellules en phase de croissance. Le nombre de cellules vivantes a été déterminé par la méthode au MTT après 1, 2, 3 et 4 jours de culture. La cytotoxicité a été mesurée et est exprimée sous forme de $CD_{50}$.

Les lignées cellulaires continues utilisées sont les suivantes :

- Cellules en suspension :

| | |
|---|---|
| MT-4<br>HUT-78<br>Molt4 (clone 8) | lignées lymphocytaires humaines à CD4 |
| U-937<br>THP-1 | lignées monocytaires humaines |

- Cellules adhérentes :

Hep-G2 : hépatocarcinome humain

Hep-2 : carcinome épidermoïde humain

Les résultats sont repris au tableau 8. Il en ressort que le dérivé 5'hémisuccinyl est moins toxique que l'AZT sur toutes les lignées cellulaires testées.

Tableau 8 : Cytotxicité in vitro de l'AZT 5-hémisuccinyl sur différentes lignées cellulaires

| Lignée cellulaire | Produit | Jour 1 | | Jour 2 | | Jour 3 | | Jour 4 | |
|---|---|---|---|---|---|---|---|---|---|
| | | $CD_{50}$(µM) | Rapport | $CD_{50}$(µM) | Rapport | $CD_{50}$(µM) | Rapport | $CD_{50}$(µM) | Rapport |
| MT-4 | AZT | > 1000 | 1 | 149 | 1 | 132 | 1 | 148 | 1 |
| | AZT-5'HS | > 12000 | - | 3090 | 21 | 2050 | 16 | 2000 | 13 |
| HUT-78 | AZT | 5640 | 1 | 3560 | 1 | 3910 | 1 | 3410 | 1 |
| | AZT-5'HS | > 12000 | > 2 | 8380 | 2.4 | 9010 | 2.3 | 8000 | 2.4 |
| Molt-4(clone 8) | AZT | > 1000 | 1 | > 1000 | 1 | 504 | 1 | 409 | 1 |
| | AZT-5'HS | > 20000 | - | 5290 | < 5 | 3130 | 6 | 3200 | 8 |
| U-937 | AZT | > 1000 | 1 | 82 | 1 | 97 | 1 | 100 | 1 |
| | AZT-5'HS | 8350 | < 8.4 | 2260 | 28 | 2210 | 23 | 2300 | 23 |
| THP-1 | AZT | > 1000 | 1 | 362 | 1 | 173 | 1 | 209 | 1 |
| | AZT-5'HS | > 20000 | - | 6880 | 19 | 4380 | 25 | 3680 | 18 |
| Hep-G2 | AZT | ND | - | 1240 | 1 | 1340 | 1 | 2130 | 1 |
| | AZT-5'HS | ND | - | 500 | 0.4 | 4180 | 3 | 14240 | 7 |
| HEp-2 | AZT | 6720 | 1 | 6070 | 1 | 4830 | 1 | 4760 | 1 |
| | AZT-5'HS | > 20000 | > 3 | >20000 | > 3 | > 20000 | > 4 | >20000 | > 4 |

AZT-5'HS = AZT-5'hémisuccinyl

ND = non déterminé

$$\text{Rapport} = \frac{CD_{50}\ \text{AZT-5'HS}}{CD_{50}\text{AZT}}$$

1.2) Toxicité sur cellules de la moëlle osseuse

Des cellules ont été isolées à partir d'un prélèvement de moëlle osseuse chez un donneur sain. Elles ont été mises en culture en milieu semi-solide (agar) additionné de Granulocyte-Macrophage-Colony Stimulating Factor (GMCSF) en présence d'AZT et d'AZT 5'hémisuccinyl à différentes concentrations. Le nombre de cellules ayant donné naissance à une colonie a été déterminé par comptage de colonies au jour 7.

La toxicité du dérivé est de l'ordre de 10 fois plus faible que celle de l'AZT.

2) Toxicité in vivo du dérivé

L'AZT et son dérivé 5'hémisuccinyl ont été administrés par voie infra-péritonéale à de souriceaux NMRI âgés de 5 jours (100 µl/g de poids corporel). Les animaux ont été contrôlés tous les jours selon 2 critères : survie et poids pendant 25 jours.

Les résultats sont repris au tableau 9. Les souriceaux traités n'ont pas montre de différence significative dans la prise de poids par rapport aux contrôles.

Tableau 9

Toxicité in vivo de l'AZT et de l'AZT 5'hémisuccinyl administrés intra-péritonéalement à des souriceaux NMRI de 5 jours

| Produit | Dose (équivalent AZT) g/kg poids corporel | Survivants au jour 25 (mortalité/ jour) % |
|---|---|---|
| AZT | 1 (1) | 0 (100 % au jour 1) |
| | 2 (2) | 0 (100 % au jour 1) |
| AZT-5'hémisuccinyl | 1 (0.73) | 100 |
| | 2 (1.46) | 100 |
| | 3 (2.2) | 50 (50 % au jour 1) |
| Contrôle | - - | 100 |

**Revendications**

1. Composés chimiques dérivés de la 3'azido-3' déoxythymidine et leurs sels pharmaceutiquement acceptables, caractérisés en ce qu'ils répondent à la formule générale I

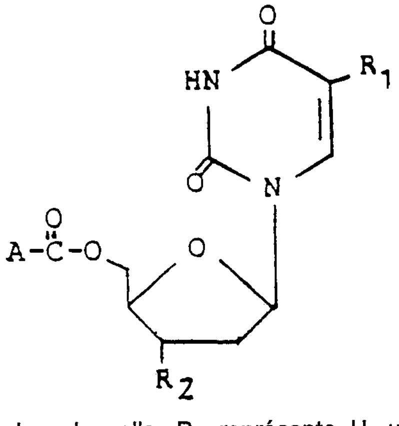

I

dans laquelle. $R_1$ représente H, un radical alkyle inférieur, un radical alcoxy inférieur, un radical hydroxyalcoyle inférieur ou un halogène.

. $R_2$ est $N_3$ ou un radical -CN et

. A représente un radical hydrocarboné éventuellement substitué, portant au moins une fonction polaire, notamment carboxylique, sulfonique, ou amine éventuellement protégée, ou telles que leurs fonctions dérivées par substitution d'hydrogène par un radical hydrocarboné inférieur qui peut, pour accentuer le caractère polaire, être raccordé à A en formant un hétérocycle, A n'étant pas le groupe hémisuccinyl.

2. Composés chimiques selon la revendication 1, caractérisés en ce que $R_1$ est un radical $-CH_3$ et $R_2$ est $N_3$.

3. Composés selon l'une des revendications 1 ou 2, caractérisés en ce que

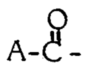 représente 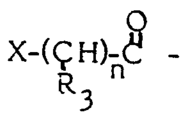

où n est un nombre entier compris entre 1 et 5,
$R_3$ peut être identique ou différent et représente H ou un radical hydrocarboné, tel qu'un alkyle, un aralkyle, un alcoxyalkyle, éventuellement substitués ; ou la chaîne latérale en alpha de la fonction amino d'un acide aminé entrant dans la constitution des protéines naturelles ; ou un groupe NHR avec R = H, alkyl, alcoxycarbonyl, formyl ou acyl ; ou avec un autre $R_3$ peut représenter simplement une liaison pour former une double liaison ; et
X= COOH, $SO_3H$, ou $NH_2$ éventuellement protégée.

4. Composés selon l'une des revendications 1 à 3, caractérisés en ce que la fonction polaire X = COOH, ou $NH_2$ éventuellement protégée.

5. Composés selon la revendication 3, caractérisés par le fait que $R_3$ = H, X = COOH.

6. Composés selon la revendication 3, caractérisés en ce que

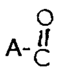 représente

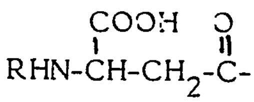 ou

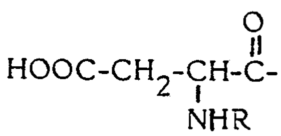

avec R = H, alkyl, formyl, acyl ou alcoxycarbonyl.

7. Composés selon l'une des revendications 1 ou 2, caractérisés en ce que 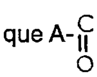 représente le reste éventuellement protégé d'un acide aminé A-COOH, notamment d'un acide aminé entrant dans la constitution des produits naturels.

8. Composés selon la revendication 7 ou 3, caractérisés en ce que dans la formule

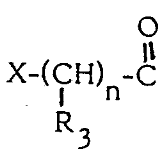

n = 1, X = $NH_2$ éventuellement protégé et $R_3$ représente la chaîne latérale en alpha de la fonction amino d'un acide aminé entrant dans la constitution des protéines naturelles.

9. Procédé de synthèse des composés selon l'une des revendications 1 à 8, caractérisé en ce que l'on fait réagir un composé de formule II suivante.

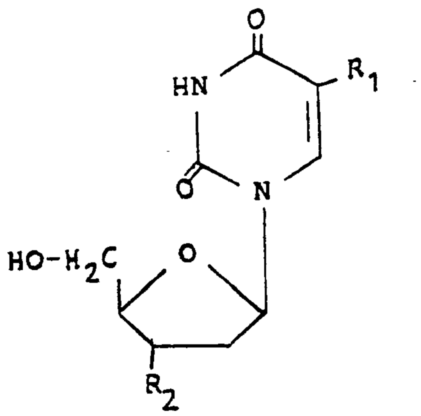

II.

avec un réactif choisi dans le groupe constitué par les esters d'acide carboxylique de formule A-COOH, les sels halogénures de cet acide et les anhydrides d'acide interne ou externe.

10. Compostition pharmaceutique, caractérisée en ce qu'elle comporte à titre de substance active l'un des composés selon l'une des revendications 1 à 8 ou le dérivé hémisuccinyl en position 5′ de l'AZT.

11. Composition pharmaceutique à toxicité réduite ou à activité thérapeutique augmentée par rapport à celles de l'AZT pour le traitement à long terme du SIDA, et notamment pour le traitement de personnes séropositives infectées par le virus du SIDA avant que la maladie ne se soit déclarée, caractérisée en ce qu'elle comporte un composé selon l'une des revendications précédentes, ou le dérivé hémisuccinyl en 5′ de l'AZT en une quantité thérapeutiquement efficace.

12. Composition pharmaceutique selon la revendication précédente, caractérisée en ce qu'elle comporte une quantité molaire réduite de substance active par rapport à la quantité molaire d'AZT nécessaire pour obtenir la même activité thérapeutique, ou supérieur par rapport à la quantité molaire d'AZT induisant une toxicité identique.

13. Utilisation des composés selon l'une des revendications 1 à 8 ou du dérivé hémisuccinyl en 5′ de l'AZT pour la préparation de compositions pharmaceutiques selon les revendications 11 ou 12.

14. Procédé pour diminuer la toxicité ou augmenter l'activité de l'ART et améliorer l'indice de sélectivité de l'AZT, caractérisé en ce qu'on effectue une estérification en position 5′ d'un composé de formule II tel que de l'AZT avec un acide approprié selon le procédé de la revendication 9 pour obtenir un ester d'acide carboxylique selon l'une des revendications 1 à 8 ou le dérivé hémisuccinyl en 5′ de l'AZT.

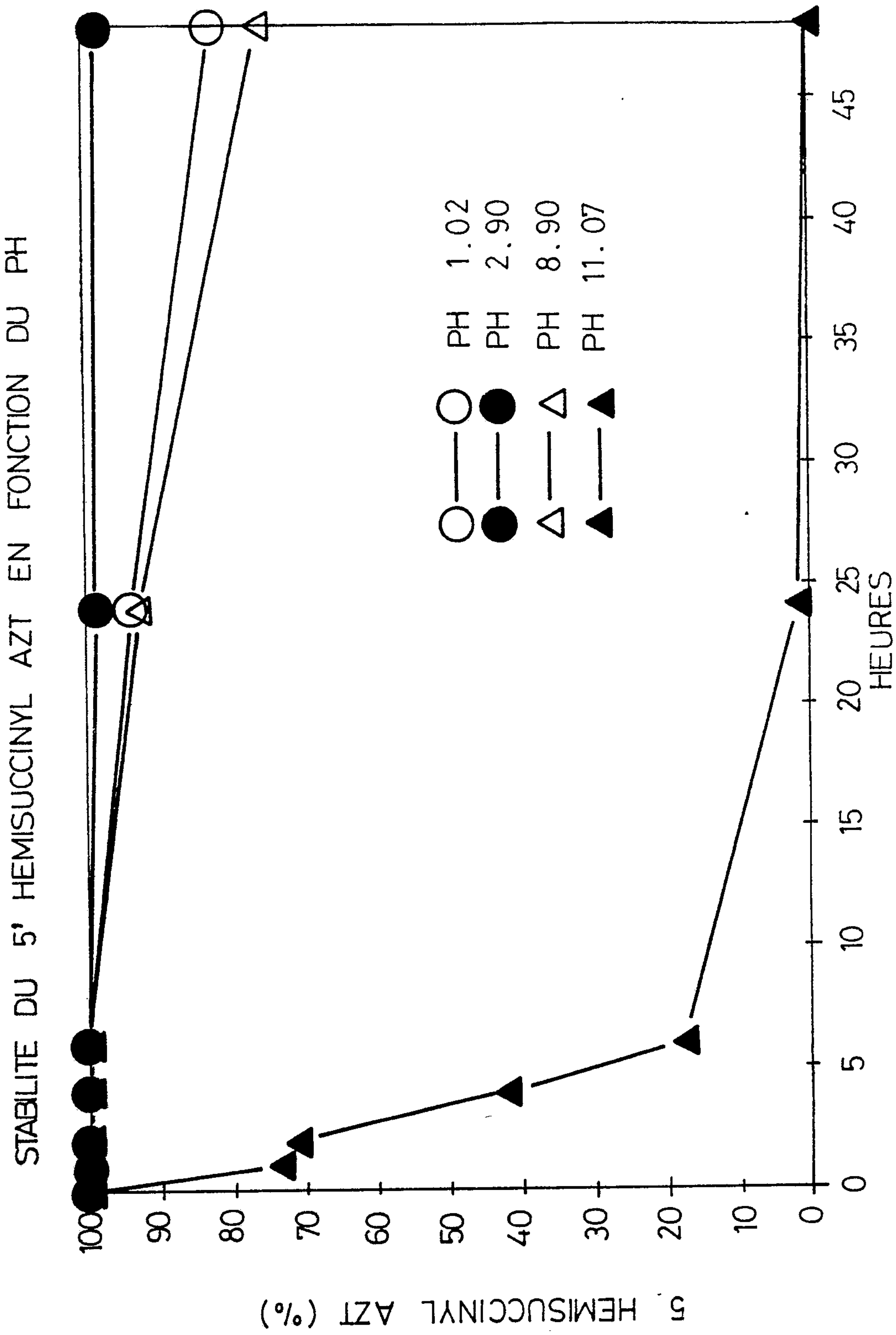
STABILITE DU 5' HEMISUCCINYL AZT EN FONCTION DU PH
5' HEMISUCCINYL AZT (%)
100
90
80
70
60
50
40
30
20
10
0
0
5
10
15
20
25
30
35
40
45
HEURES
PH 1.02
PH 2.90
PH 8.90
PH 11.07

FIG_1

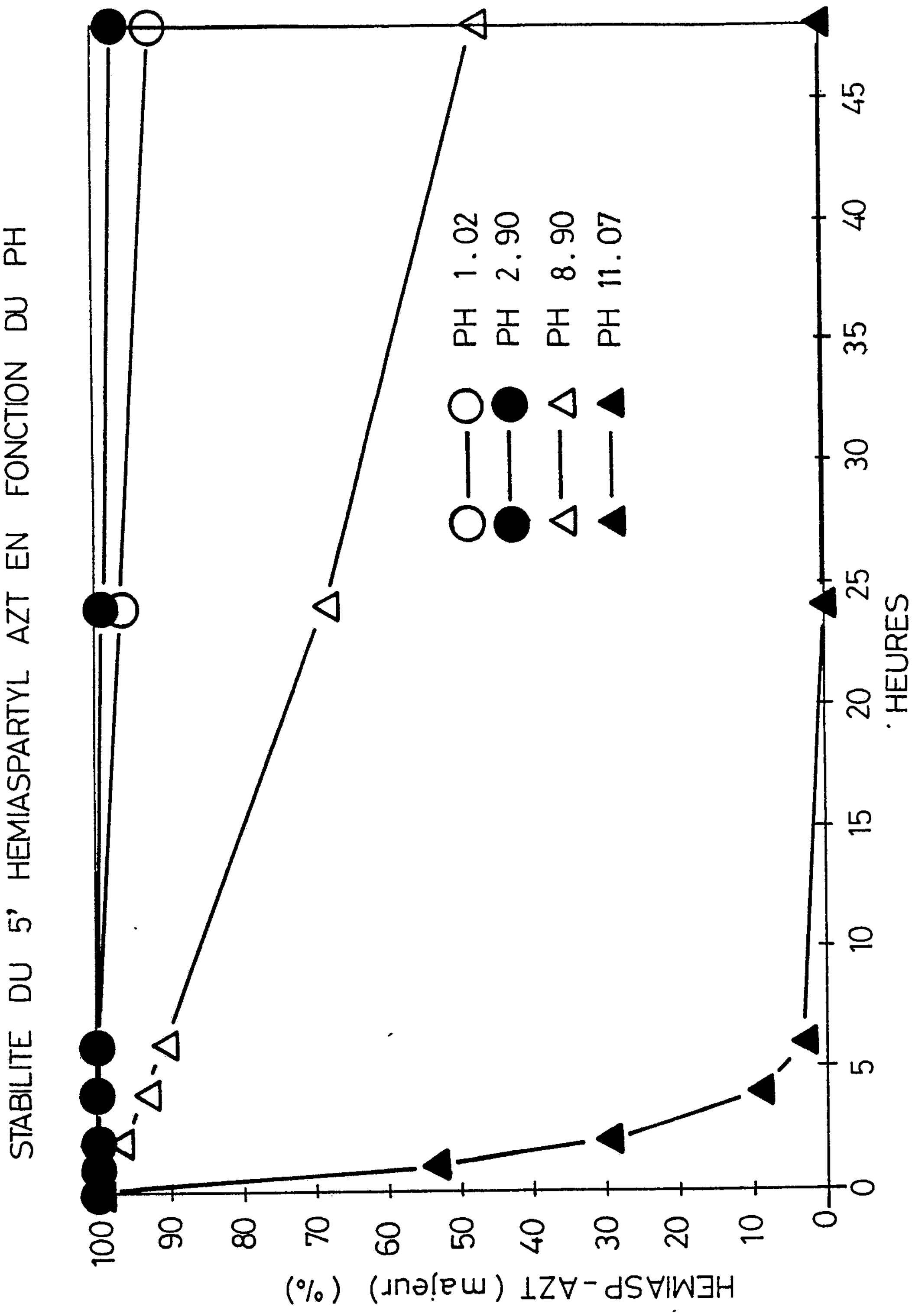
STABILITE DU 5' HEMIASPARTYL AZT EN FONCTION DU PH
PH 1.02
PH 2.90
PH 8.90
PH 11.07
HEMIASP-AZT ( majeur ) (%)
100
90
80
70
60
50
40
30
20
10
0
0
5
10
15
20
25
30
35
40
45
HEURES

FIG.2

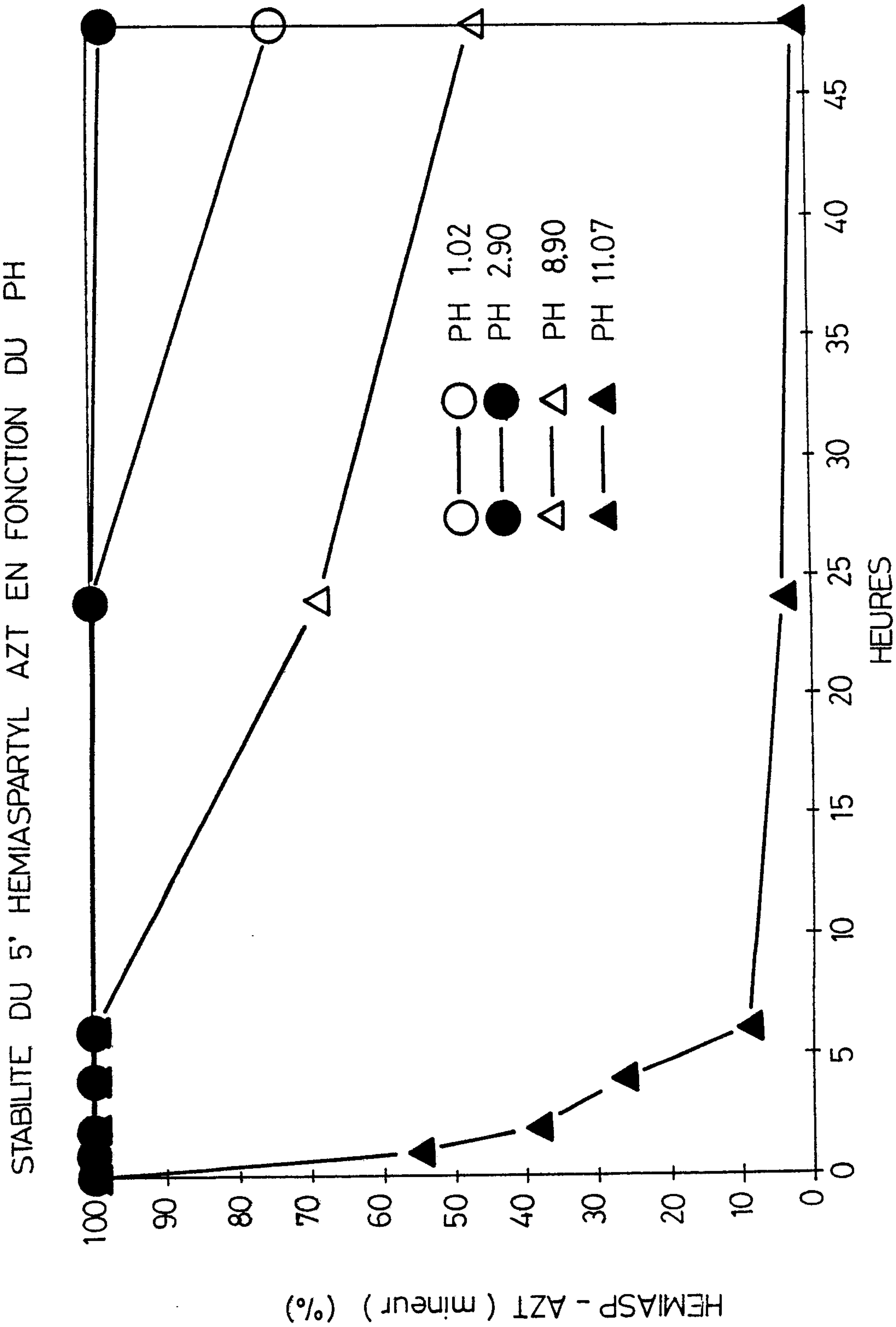
STABILITE DU 5' HEMIASPARTYL AZT EN FONCTION DU PH
PH 1.02
PH 2.90
PH 8,90
PH 11.07
HEMIASP - AZT ( mineur ) (%)
100
90
80
70
60
50
40
30
20
10
0
0
5
10
15
20
25
30
35
40
45
HEURES

FIG_3

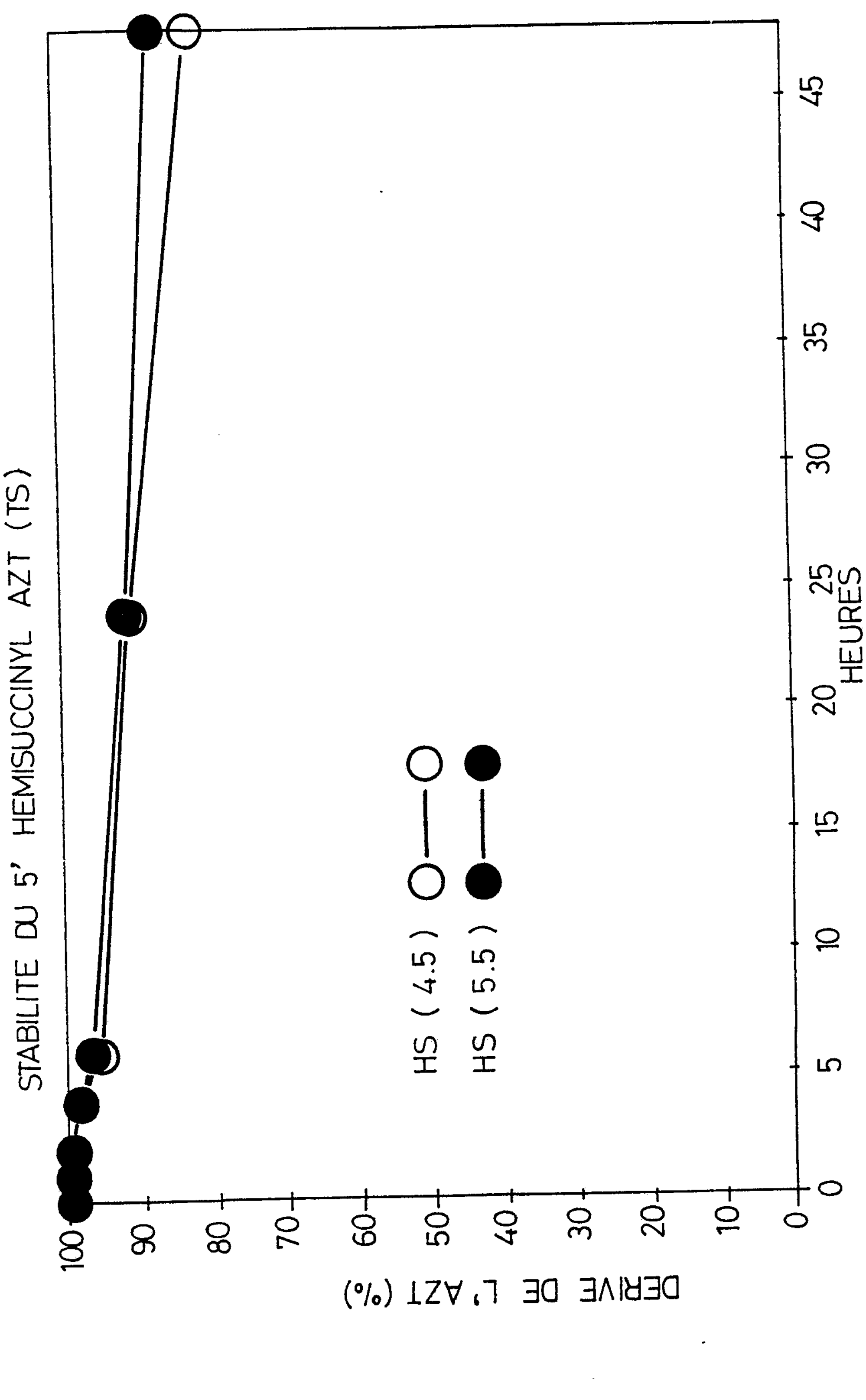


TS = ENZYMES LYSOSOMIALES

FIG_4

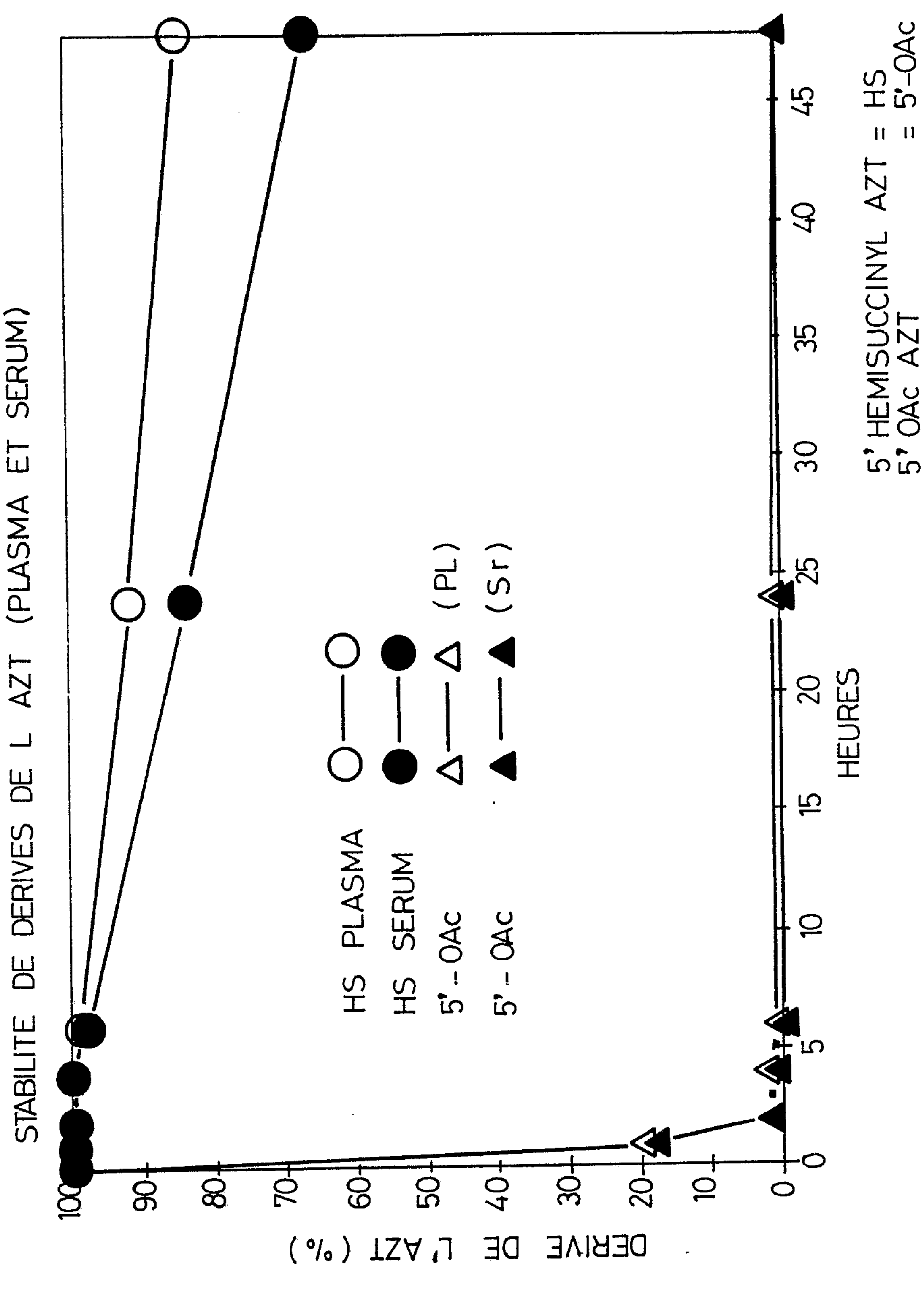
STABILITE DE DERIVES DE L AZT (PLASMA ET SERUM)
DERIVE DE L'AZT (%)
100
90
80
70
60
50
40
30
20
10
0
0
5
10
15
20
25
30
35
40
45
HEURES
HS PLASMA
HS SERUM
5' - OAc (PL)
5' - OAc (Sr)
5' HEMISUCCINYL AZT = HS
5' OAc AZT = 5'-OAc

FIG_5

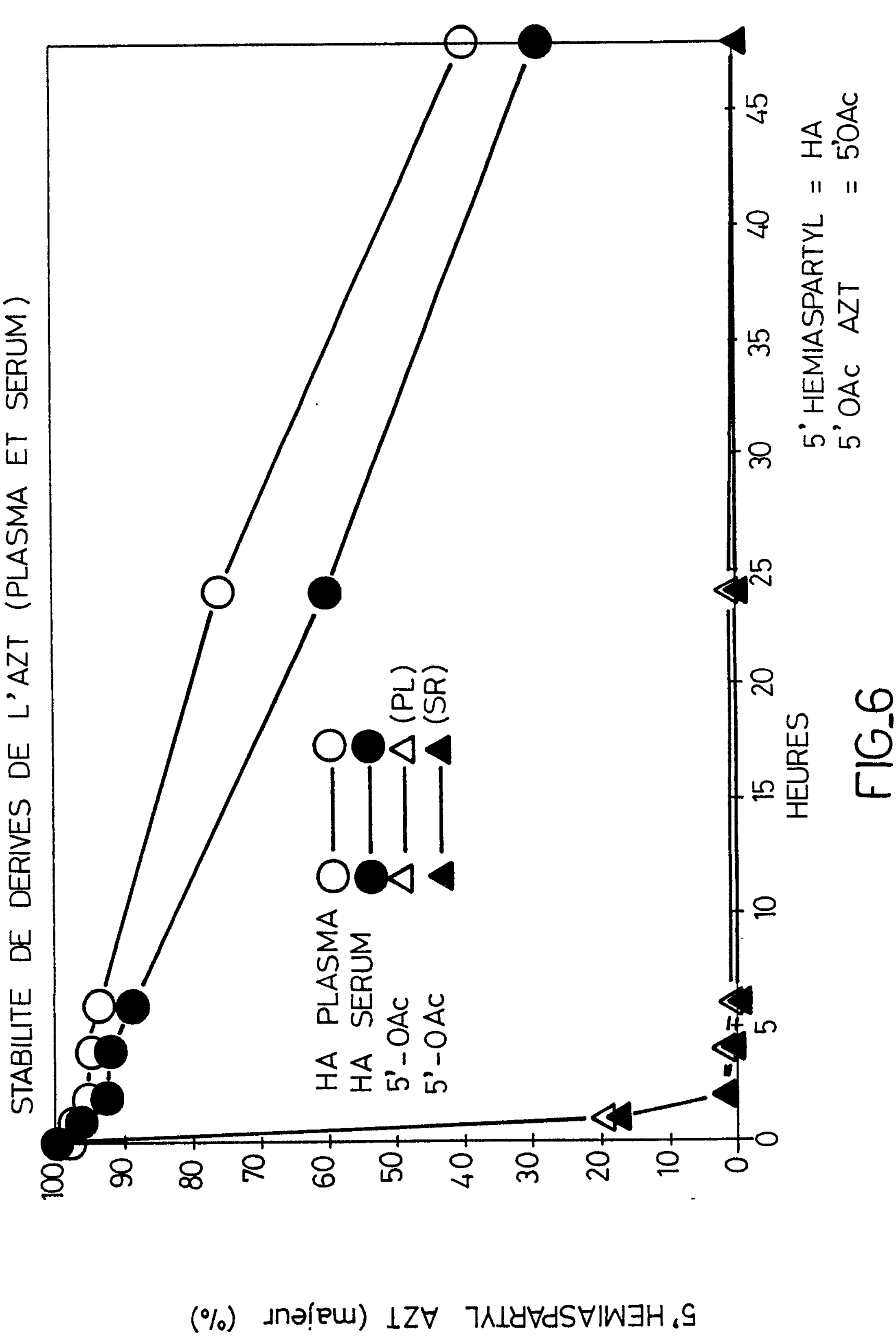
STABILITE DE DERIVES DE L'AZT (PLASMA ET SERUM)
HA PLASMA
HA SERUM
5'-OAc (PL)
5'-OAc (SR)
5' HEMIASPARTYL = HA
5' OAc AZT = 5'OAc
HEURES
0
5
10
15
20
25
30
35
40
45
100
90
80
70
60
50
40
30
20
10
0
5' HEMIASPARTYL AZT (majeur) (%)

FIG_6

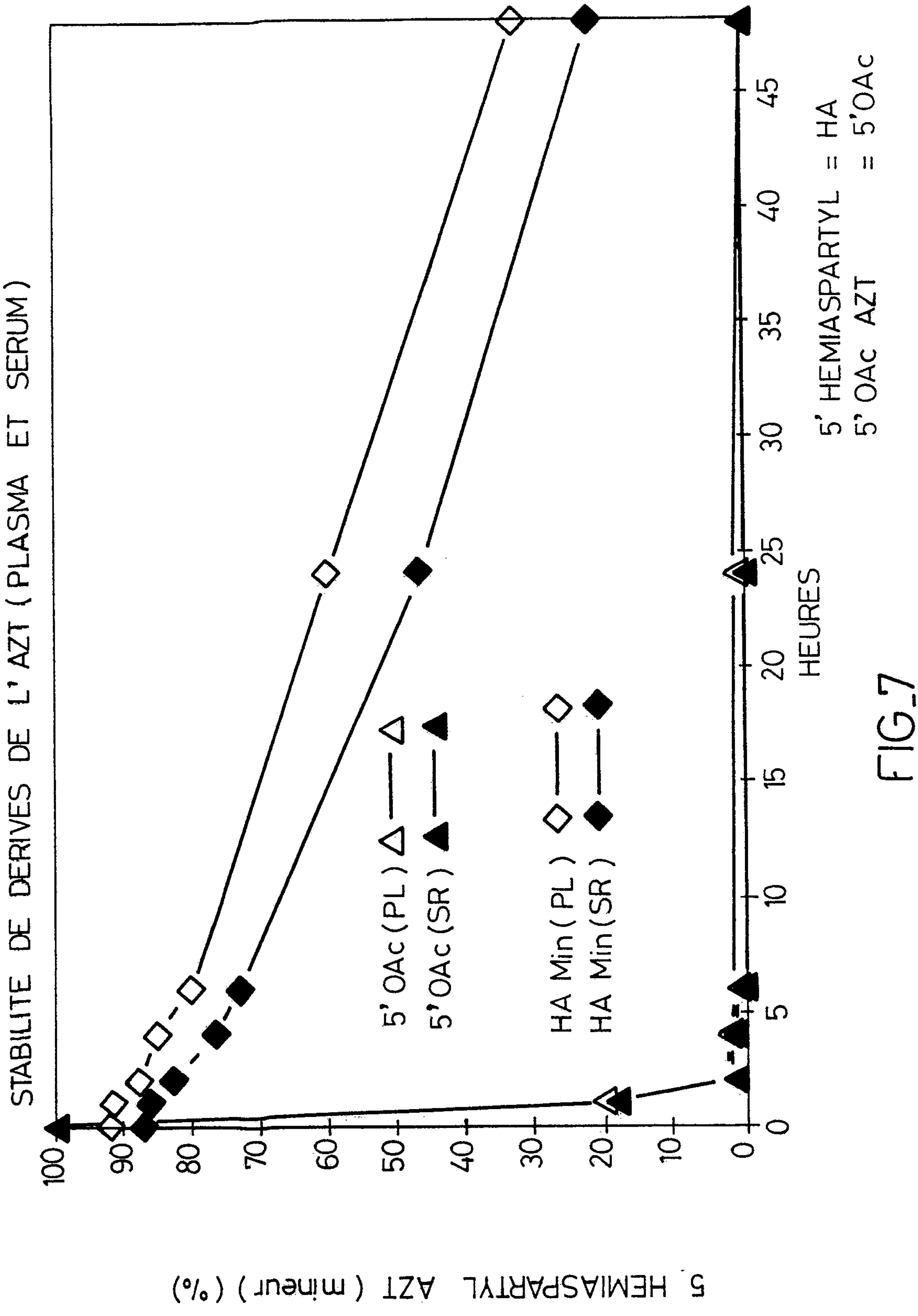

STABILITE DE DERIVES DE L' AZT ( PLASMA ET SERUM )
5' HEMIASPARTYL AZT ( mineur ) (%)
100
90
80
70
60
50
40
30
20
10
0
0
5
10
15
20
25
30
35
40
45
HEURES
5' OAc ( PL )
5' OAc ( SR )
HA Min ( PL )
HA Min ( SR )
5' HEMIASPARTYL = HA
5' OAc AZT = 5'OAc


FIG_7

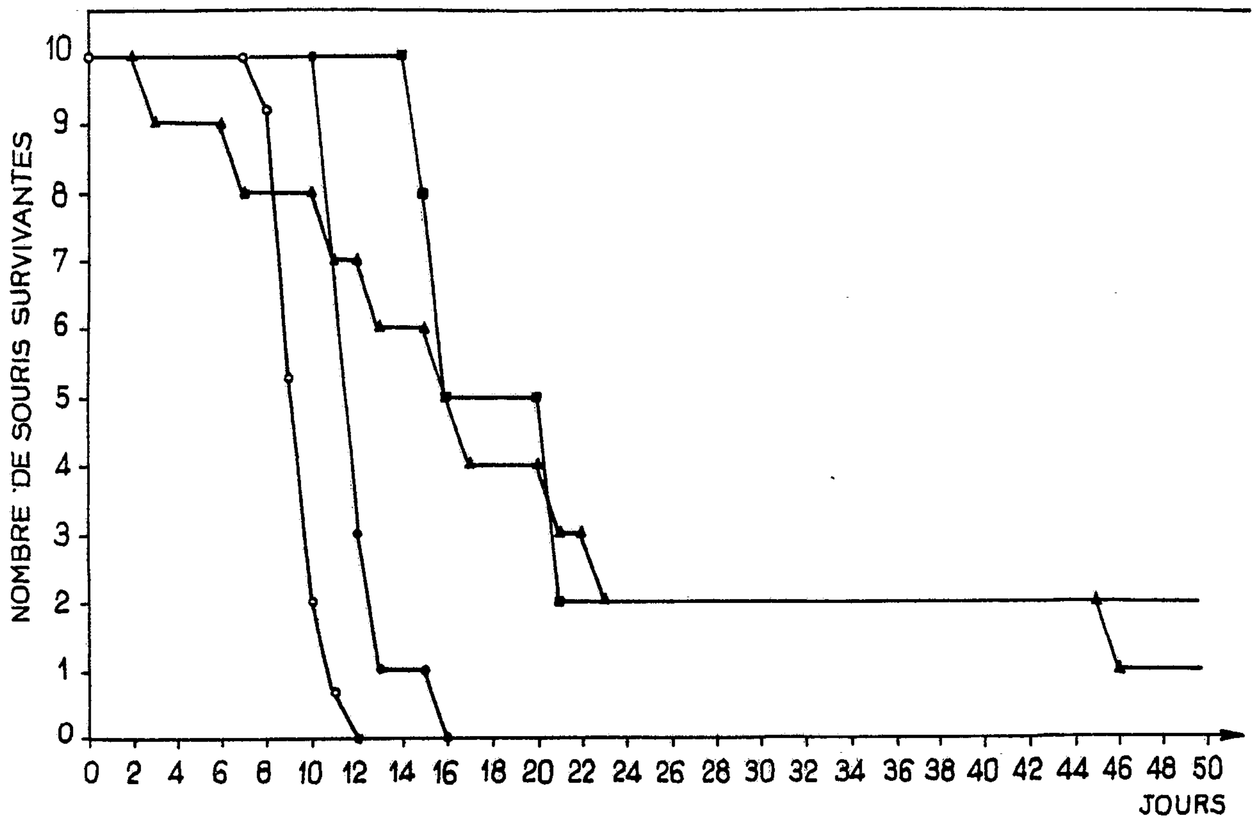
NOMBRE DE SOURIS SURVIVANTES
10
9
8
7
6
5
4
3
2
1
0
0 2 4 6 8 10 12 14 16 18 20 22 24 26 28 30 32 34 36 38 40 42 44 46 48 50
JOURS

FIG_8

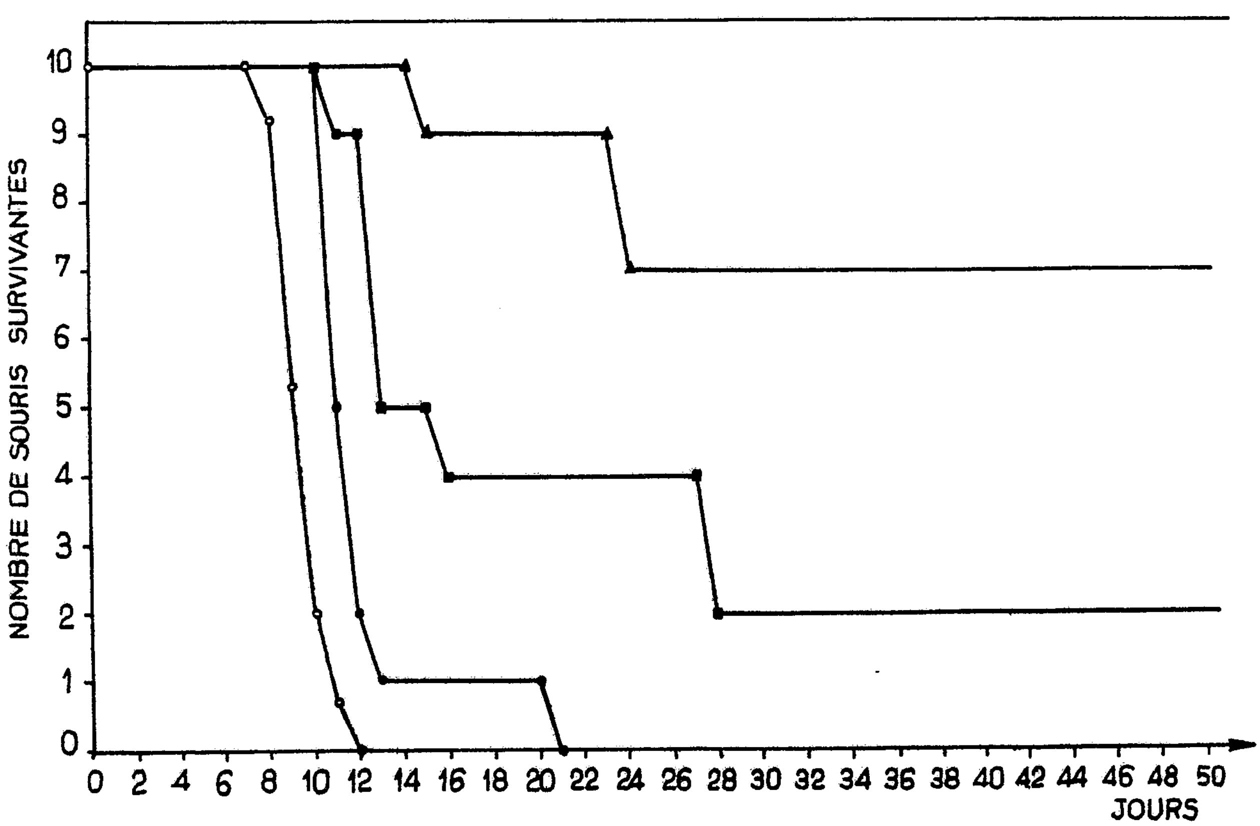
NOMBRE DE SOURIS SURVIVANTES
10
9
8
7
6
5
4
3
2
1
0
0 2 4 6 8 10 12 14 16 18 20 22 24 26 28 30 32 34 36 38 40 42 44 46 48 50
JOURS

FIG_9

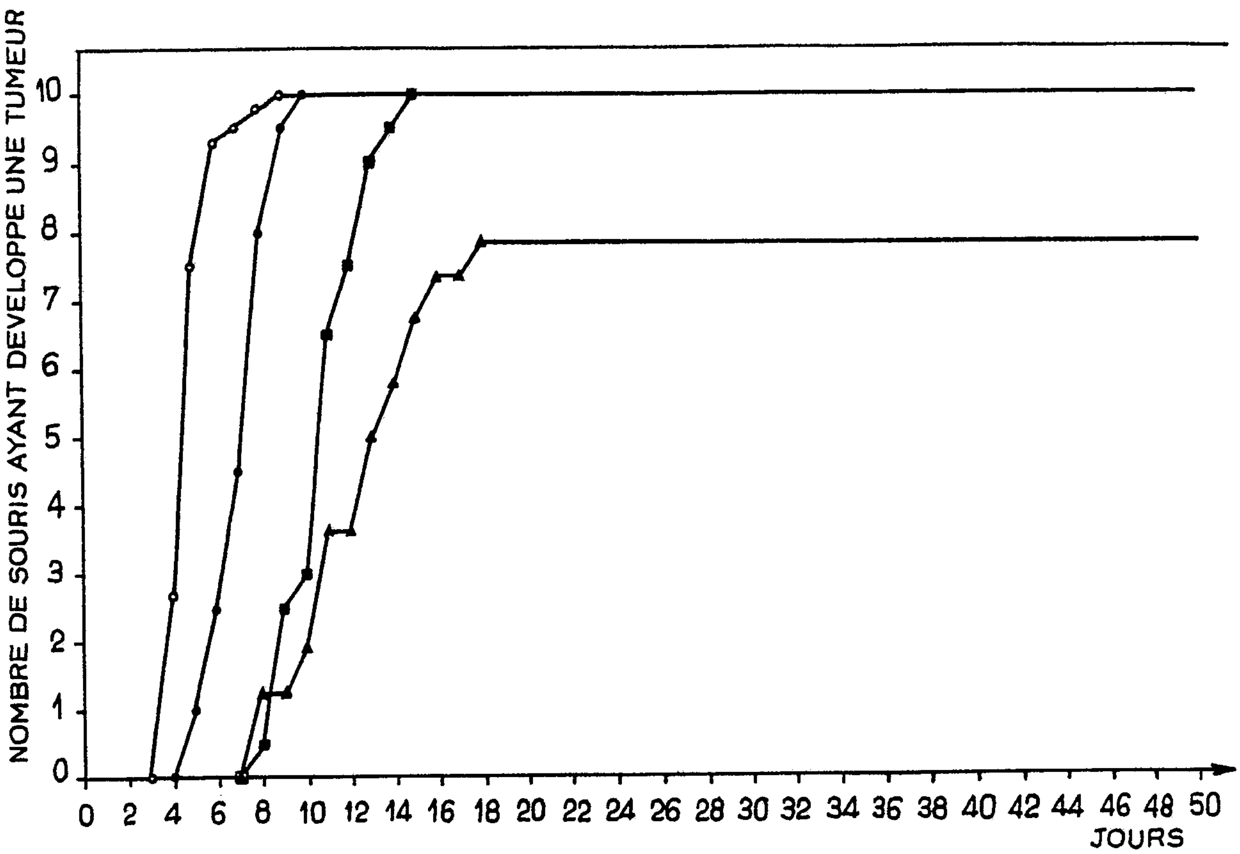
NOMBRE DE SOURIS AYANT DEVELOPPE UNE TUMEUR
0
1
2
3
4
5
6
7
8
9
10
0 2 4 6 8 10 12 14 16 18 20 22 24 26 28 30 32 34 36 38 40 42 44 46 48 50
JOURS

FIG_10

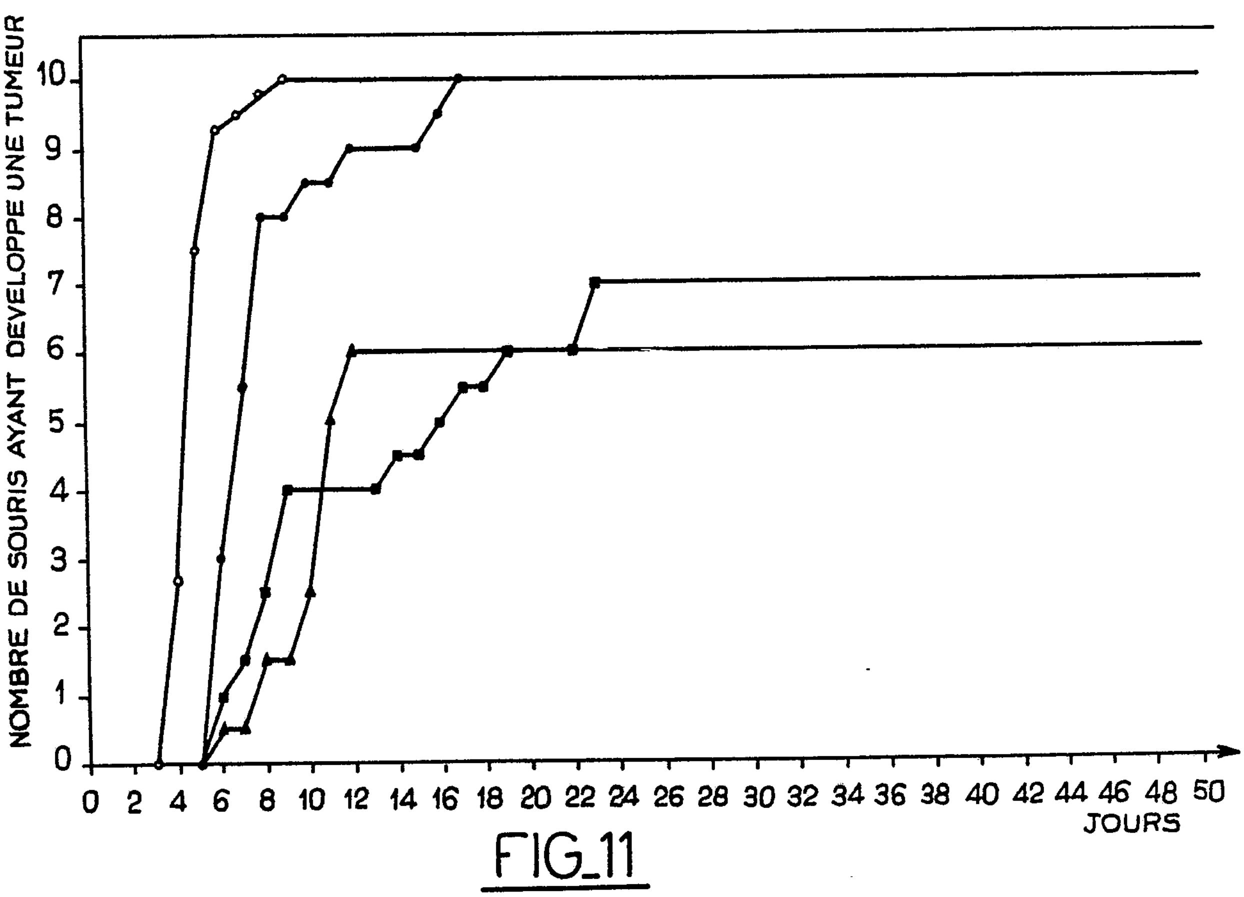
NOMBRE DE SOURIS AYANT DEVELOPPE UNE TUMEUR
0
1
2
3
4
5
6
7
8
9
10
0 2 4 6 8 10 12 14 16 18 20 22 24 26 28 30 32 34 36 38 40 42 44 46 48 50
JOURS

FIG_11